# EUROPÄISCHE PATENTSCHRIFT

(11) **EP 3 635 728 B1**
(45) Veröffentlichungstag und Bekanntmachung des Hinweises auf die Patenterteilung: **27.11.2024**
(21) Anmeldenummer: 17729456.8
(22) Anmeldetag: 08.06.2017
(51) Int. Cl.: G16H 10/20, G16H 80/00, G06Q 10/0631, G06Q 10/1053

(54) **VERFAHREN ZUR PATIENTENREKRUTIERUNG UND PATIENTENREKRUTIERUNGSSYSTEM**
METHOD FOR RECRUITING PATIENTS, AND PATIENT RECRUITMENT SYSTEM
PROCÉDÉ DE RECRUTEMENT DE PATIENT ET SYSTÈME DE RECRUTEMENT DE PATIENT

(43) Veröffentlichungstag der Anmeldung: 15.04.2020
(73) Patentinhaber: Clinerion Ltd., 4051 Basel (CH)
(72) Erfinder: WALTER, Andreas, 76532 Baden-Baden (DE); ARONSKY, Dominik, 8803 Rüschlikon (CH); CLAESSON, Ulf, 8907 Wettswil (CH)
(74) Vertreter: Daub, Thomas
(86) Internationale Anmeldenummer: PCT/EP2017/064024
(87) Internationale Veröffentlichungsnummer: WO 2018/224156

(56) Entgegenhaltungen:
- WO-A1-2016/171376
- CN-A- 101 707 056
- CN-A- 105 070 272
- US-A1- 2005 182 665
- US-A1- 2014 316 793
- US-A1- 2016 029 964

## Beschreibung

### Stand der Technik

Die Erfindung betrifft ein Verfahren zur Patientenrekrutierung nach dem Oberbegriff des Anspruchs 1 sowie ein Patientenrekrutierungssystem nach dem Oberbegriff des Anspruchs 8.

Aus dem Stand der Technik, z.B. den veröffentlichten US-Patentanmeldungen US 2005/182665 A1 und US 2014/316793 A1, sind Patientendatenbanken bekannt, in denen Patientendatensätze hinterlegt sind, die zu einer Rekrutierung von Patienten für klinische Studien herangezogen werden.

Die Aufgabe der Erfindung besteht insbesondere darin, vorteilhafte Eigenschaften hinsichtlich einer Patientenrekrutierung zu erzielen. Zudem besteht eine Aufgabe der Erfindung insbesondere darin, eine hohe Qualität und/oder Effizienz zu erzielen. Außerdem besteht eine Aufgabe der Erfindung insbesondere darin, ein Auswählen möglicher Teilnehmer für klinische Studien mit einer hohen Güte zu erzielen. Die Aufgabe wird erfindungsgemäß durch die Merkmale der Ansprüche 1 und 8 gelöst, während vorteilhafte Ausgestaltungen und Weiterbildungen der Erfindung den Unteransprüchen entnommen werden können.

### Vorteile der Erfindung

Die Erfindung geht aus von einem Verfahren, insbesondere einem Patientenrekrutierungsverfahren, zur Patientenrekrutierung für eine Studie, insbesondere für zumindest eine klinische Studie, wobei in zumindest einem Abgleichschritt zumindest ein auf einem Patientendatenserver hinterlegter Patientenparameter zumindest eines Patienten mit zumindest einem auf einem Studiendatenserver hinterlegten Rekrutierungsparameter zumindest einer Studie maschinell, und insbesondere automatisiert, abgeglichen wird, und wobei in zumindest einem Bewertungsschritt zumindest ein Eignungsparameter einer Eignung des Patienten für die Studie in Abhängigkeit von dem Patientenparameter und dem Rekrutierungsparameter maschinell, und insbesondere automatisiert, ermittelt und auf zumindest einem Server, insbesondere einem Patientenrekrutierungsserver, hinterlegt wird.

Erfindungsgemäß wird in zumindest einem Zuordnungsschritt zumindest eine Zuordnung zwischen dem Patienten und der Studie in Abhängigkeit von dem Eignungsparameter durchgeführt und zumindest eine Zuordnungsinformation auf zumindest einem Server, insbesondere dem Patientenrekrutierungsserver, hinterlegt.

Durch das erfindungsgemäße Verfahren können vorteilhafte Eigenschaften hinsichtlich einer Patientenrekrutierung, insbesondere hinsichtlich deren Effizienz und/oder Qualität, erzielt werden. Vorteilhaft kann eine hohe Güte einer Auswahl geeigneter Patienten erzielt werden. Außerdem können vorteilhaft geeignete Patienten zu einer Teilnahme an klinischen Studien animiert und/oder ein Rekrutierungsaufwand verringert werden. Zudem kann vorteilhaft ein Patient und/oder ein dem Patienten zugeordnetes klinisches Personal über geeignete Studien informiert und zu einem aktiven Verhalten angeregt werden. Ferner kann ein Bewusstsein von Patienten für klinische Studien erzeugt und hierdurch ein Aufwand für eine Patientenrekrutierung reduziert werden. Des Weiteren kann eine hohe Kosteneffizienz bei einer Patientenrekrutierung erzielt werden. Insbesondere können Patienten für Studien im Zusammenhang mit einem Nachweis einer Wirksamkeit von insbesondere zukünftig zuzulassenden Wirkstoffen und/oder Behandlungsmethoden effizient rekrutiert werden.

Zudem umfasst die Erfindung ein Patientenrekrutierungssystem, insbesondere zur Rekrutierung von Patienten für Studien, vorteilhaft für klinische Studien, insbesondere vorgesehen zur Durchführung des Verfahrens zur Patientenrekrutierung, mit zumindest einem Patientenrekrutierungsserver, der zur Ausführung zumindest eines Abgleichprozesses zum Abgleich zumindest eines Patientenparameters zumindest eines Patienten mit zumindest einem Rekrutierungsparameter zumindest einer Studie sowie zur Ausführung zumindest eines Bewertungsprozesses zur Generierung zumindest eines Eignungsparameters einer Eignung des Patienten für die Studie in Abhängigkeit von dem Patientenparameter und dem Rekrutierungsparameter vorgesehen ist.

Es wird vorgeschlagen, dass der Patientenrekrutierungsserver zur Ausführung zumindest eines Zuordnungsprozesses zur Erzeugung zumindest einer Zuordnung zwischen der Studie und dem Patienten in Abhängigkeit von dem Eignungsparameter vorgesehen ist.

Durch die erfindungsgemäße Ausgestaltung des Patientenrekrutierungssystems können vorteilhafte Eigenschaften hinsichtlich einer Patientenrekrutierung, insbesondere hinsichtlich deren Effizienz und/oder Qualität, erzielt werden. Vorteilhaft kann eine hohe Güte einer Auswahl geeigneter Patienten erzielt werden. Außerdem können vorteilhaft geeignete Patienten zu einer Teilnahme an klinischen Studien animiert und/oder ein Rekrutierungsaufwand verringert werden. Zudem kann vorteilhaft ein Patient über geeignete Studien informiert und zu einem aktiven Verhalten angeregt werden. Ferner kann ein Bewusstsein von Patienten für klinische Studien erzeugt und hierdurch ein Aufwand für eine Patientenrekrutierung reduziert werden. Des Weiteren kann eine hohe Kosteneffizienz bei einer Patientenrekrutierung erzielt werden.

Insbesondere beinhaltet der Abgleichprozess eine, insbesondere maschinelle, Durchführung des Abgleichschritts und/oder der Bewertungsprozess eine, insbesondere maschinelle, Durchführung des Bewertungsschritts und/oder der Zuordnungsprozess eine, insbesondere maschinelle, Durchführung des Zuordnungsschritts. Unter einem "Prozess" soll in diesem Zusammenhang insbesondere eine maschinelle Routine und/oder eine maschinelle Teilprogramm- oder Programmausführung, die insbesondere mittels zumindest einer Recheneinheit durchführbar ist und/oder in zumindest einem Betriebszustand durchgeführt wird, verstanden werden. Unter einer "Recheneinheit" soll insbesondere eine Einheit mit einem Informationseingang, einer Informationsverarbeitung und einer Informationsausgabe verstanden werden. Vorteilhaft weist die Recheneinheit zumindest einen Prozessor, einen Speicher, ein oder mehrere Ein- und Ausgabemittel, weitere elektrische Bauteile, ein Betriebsprogramm, Regelroutinen, Steuerroutinen und/oder Berechnungsroutinen auf.

Vorzugsweise ist das Verfahren zu einer Patientenrekrutierung vorgesehen. Besonders bevorzugt ist das Verfahren und/oder das Patientenrekrutierungssystem dazu vorgesehen, Patienten für klinische Studien der Phase III zu rekrutieren. Es ist daneben aber auch eine Rekrutierung von Patienten für klinische Studien einer anderen Phase und/oder für andere Arten von Studien denkbar. insbesondere kann die Patientenrekrutierung eine Rekrutierung von Patienten im medizinischen und/oder veterinärmedizinischen Bereich, vorteilhaft für entsprechende, insbesondere klinische, Studien, umfassen. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt. Darunter, dass ein Verfahren zu einem Zweck "vorgesehen" ist, soll insbesondere verstanden werden, dass das Verfahren zumindest einen Verfahrensschritt beinhaltet, der speziell auf den Zweck abzielt und/oder dass das Verfahren gezielt auf den Zweck gerichtet ist und/oder dass das Verfahren einer Erfüllung des Zwecks dient und auf diese Erfüllung hin zumindest teilweise optimiert ist. Darunter, dass ein Verfahrensschritt zu einem Zweck "vorgesehen" ist, soll insbesondere verstanden werden, dass der Verfahrensschritt speziell auf den Zweck abzielt und/oder dass der Verfahrensschritt gezielt auf den Zweck gerichtet ist und/oder dass der Verfahrensschritt einer Erfüllung des Zwecks dient und auf diese Erfüllung hin zumindest teilweise optimiert ist.

Unter einem "Patientendatenserver" soll insbesondere ein Server verstanden werden, auf welchem Patientendatensätze hinterlegbar und/oder hinterlegt sind. Der Patientendatenserver kann als ein dedizierter Server ausgebildet sein, alternativ oder zusätzlich aber auch als ein virtueller Server und/oder ein Cloud-Server und/oder ein Shared Server und/oder als zumindest ein Teil eines Content Delivery Networks oder ein anderer geeigneter Server. Vorzugsweise ist der Patientenparameter zumindest ein Teil zumindest eines, insbesondere elektronischen, Patientendatensatzes. Insbesondere kann der Patientendatensatz eine Mehrzahl unterschiedlicher Patientenparameter umfassen. Der Patientenparameter umfasst insbesondere zumindest einen Wert und/oder zumindest eine Kenngröße, die zumindest ein Merkmal des Patienten und insbesondere dessen Gesundheitszustand und/oder dessen Körper kennzeichnet, beispielsweise zumindest ein Alter, insbesondere ein Mindestalter und/oder ein Höchstalter, eine Körpergröße, insbesondere eine Mindestgröße und/oder eine Maximalgröße, ein Gewicht, insbesondere ein Mindestgewicht und/oder ein Maximalgewicht, ein BMI, insbesondere ein minimaler BMI und/oder ein maximaler BMI, ein Geschlecht, eine Krankheitsgeschichte, einen Laborwert, einen Messwert, eine Medikationsinformation, einen Sensorwert, eine Suchtinformation, eine Gewohnheitsinformation, eine Verhaltensinformation und/oder dergleichen mehr, wobei beliebige Kombinationen denkbar sind. Es ist auch denkbar, dass der Patientenparameter und/oder der Patientendatensatz zumindest eine, insbesondere eindeutige, Patienten-ID umfasst. Insbesondere umfasst der Patientendatensatz zumindest einen Fall, der zumindest eine Diagnose und/oder zumindest eine Behandlungsinformation und/oder zumindest eine insbesondere mit der Behandlungsinformation verknüpfte Medikationsinformation und/oder zumindest einen Krankheitsverlauf oder dergleichen umfasst. Insbesondere umfasst der Patientendatensatz mehrere derartige Fälle und vorteilhaft wenigstens einen Teil einer Behandlungs- und/oder Krankheitsgeschichte des Patienten.

Unter einem "Studiendatenserver" soll insbesondere ein Server verstanden werden, auf welchem Studiendatensätze, insbesondere Datensätze zu klinischen Studien, hinterlegbar und/oder hinterlegt sind. Der Studiendatenserver kann als ein dedizierter Server ausgebildet sein, alternativ oder zusätzlich aber auch als ein virtueller Server und/oder ein Cloud-Server und/oder ein Shared Server und/oder als zumindest ein Teil eines Content Delivery Networks oder ein anderer geeigneter Server. Vorzugsweise ist der Rekrutierungsparameter zumindest ein Teil zumindest eines, insbesondere elektronischen, Studiendatensatzes, insbesondere eines Datensatzes einer klinischen Studie. Insbesondere kann der Studiendatensatz ein Datensatz einer klinischen Studie sein. Beispielsweise kann der Studiendatensatz zumindest eine Kontaktinformation, beispielsweise eines Studienverantwortlichen, und/oder zumindest eine Information über eine Studiendauer und/oder einen Studienort und/oder eine Studienart und/oder ein Studienziel oder andere derartige Metadaten umfassen. Vorzugsweise umfasst der Rekrutierungsparameter zumindest einen Wert und/oder zumindest einen Wertebereich und/oder zumindest eine Kenngröße und/oder zumindest eine Kategorisierungsinformation, insbesondere mehrere verkettete Kategorisierungsinformationen, und/oder zumindest ein Schlagwort, insbesondere eine, beispielsweise auch hierarchische, Kombination von Schlagworten, anhand dessen/derer eine Eignung eines Patienten, insbesondere unter Betrachtung des Patientenparameters, vorzugsweise maschinell ermittelbar ist. Beispielsweise kann der Rekrutierungsparameter ein Geschlecht, ein Alter, eine Körpergröße, ein Gewicht, ein BMI, eine Krankheitsgeschichte, einen Laborwert, einen Messwert, entsprechende Minimal- und/oder Maximalwerte, eine Medikamentierung, einen Sensorwert, eine Suchtinformation, eine Gewohnheitsinformation, eine Verhaltensinformation und/oder dergleichen, insbesondere in Form eines Wertebereichs und/oder in Form eines Entscheidungskriteriums wie beispielsweise einer booleschen Abfrage und/oder eines kategorialen Parameters umfassen. Zudem ist denkbar, dass der Studienparameter und/oder der Studiendatensatz zumindest eine, insbesondere eindeutige, Studien-ID umfasst.

Patientenparameter und/oder Studienparameter können hierbei grundsätzlich beispielsweise textuelle Informationen und/oder zumindest einen Bezug auf medizinische und/oder klinische Terminologien, insbesondere in Form eines Verweises auf eine entsprechende Datenbank und/oder zumindest eine Bildinformation und/oder Audioinformation und/oder Videoinformation und/oder zumindest einen Zeitstempel und/oder eine Chronologie oder dergleichen, umfassen.

Unter einem "Patientenrekrutierungsserver" soll insbesondere ein Server verstanden werden, auf welchem, insbesondere elektronische, Daten bezüglich einer Rekrutierung von bestimmten Patienten für bestimmte Studien hinterlegbar und/oder hinterlegt sind. Der Patientenrekrutierungsserver kann als ein dedizierter Server ausgebildet sein, alternativ oder zusätzlich aber auch als ein virtueller Server und/oder ein Cloud-Server und/oder ein Shared Server und/oder als zumindest ein Teil eines Content Delivery Networks oder ein anderer geeigneter Server. Vorzugsweise ist auf dem Patientenrekrutierungsserver zumindest ein Zuordnungsdatensatz und/oder zumindest ein Eignungsdatensatz hinterlegt. Es ist denkbar, dass der Eignungsdatensatz eine Mehrzahl unterschiedlicher Eignungsparameter enthält. Vorteilhaft ist der Eignungsdatensatz ein dem Patienten zugeordneter Eignungsdatensatz. Besonders vorteilhaft ist der Eignungsparameter ein der Studie und dem Patienten zugeordneter Eignungsparameter. Vorzugsweise sind auf dem Patientenrekrutierungsserver eine Mehrzahl unterschiedlicher Eignungsdatensätze hinterlegt, die insbesondere jeweils einem bestimmten Patienten zugeordnet sind und/oder die jeweils Eignungsparameter eines bestimmten Patienten für zumindest eine Studie enthalten.

Insbesondere weist der Patientendatenserver und/oder der Studiendatenserver und/oder der Patientenrekrutierungsserver zumindest eine Recheneinheit und/oder zumindest eine Schnittstelle zu einer zentralen Recheneinheit sowie bevorzugt zumindest einen elektronischen Datenspeicher zur Hinterlegung von Parametern und/oder Datensätzen oder dergleichen auf. Vorzugsweise sind auf dem Datenspeicher hinterlegte Parameter und/oder Datensätze verschlüsselt. Besonders bevorzugt werden Daten aus dem Datenspeicher lediglich in anonymisierter und/oder pseudonymisierter Form abgerufen und/oder auf diesem hinterlegt. Vorteilhaft sind der Patientendatenserver und/oder der Studiendatenserver und/oder der Patientenrekrutierungsserver mittels zumindest einer Datenverbindung zumindest zeitweise verbunden. Vorzugsweise sind der Patientendatenserver und/oder der Studiendatenserver und/oder der Patientenrekrutierungsserver zumindest teilweise einteilig und/oder identisch ausgebildet. Insbesondere ist denkbar, dass der Patientenrekrutierungsserver den Patientendatenserver und/oder den Studiendatenserver umfasst und/oder ausbildet. Es ist weiterhin denkbar, dass der Patientendatenserver und/oder der Studiendatenserver und/oder der Patientenrekrutierungsserver an unterschiedlichen Orten oder an demselben Ort angeordnet und/oder Teil einer Cloud sind. Vorteilhaft sind der Patientendatenserver und/oder der Studiendatenserver und/oder der Patientenrekrutierungsserver in ein Intranet einer Gesundheitsinstitution, beispielsweise eines Krankenhauses, einer Klinik, eines Ärztezentrums oder dergleichen, integriert und/oder insbesondere lediglich zu Wartungszwecken mit einem externen Netzwerk verbindbar, sodass vorteilhaft eine hohe Sicherheit und/oder eine Anonymität von personenbezogenen Daten gewährleistet werden kann.

Insbesondere umfasst der Abgleichschritt einen maschinellen Vergleich des Patientenparameters mit dem Rekrutierungsparameter und/oder zumindest ein maschinelles Aufrufen und/oder Abfragen des Patientenparameters und des Rekrutierungsparameters. Vorzugsweise ist der Patientenrekrutierungsserver dazu vorgesehen, den Abgleichschritt durchzuführen. insbesondere ist denkbar, dass in dem Abgleichschritt zumindest ein Patientendatensatz und/oder zumindest ein Studiendatensatz abgefragt und/oder zumindest temporär gespeichert werden, insbesondere zur Vorbereitung des Bewertungsschritts. Vorzugsweise umfasst der Abgleichschritt einen Vergleich eines Satzes von Patientenparametern mit einer bestimmten logischen Verknüpfung mehrerer Rekrutierungsparameter, die beispielsweise hierarchisch und/oder zumindest teilweise als Einschluss- und/oder Ausschlusskriterien zur Formulierung der logischen Verknüpfung herangezogen werden. Die logische Verknüpfung wird vorteilhaft maschinell erstellt und/oder zur maschinellen Erstellung zumindest eines maschinell ausführbaren Entscheidungs- und/oder Bewertungsprozesses herangezogen.

Vorzugsweise umfasst der Bewertungsschritt zumindest eine maschinelle Berechnung und/oder Bestimmung des Eignungsparameters, beispielsweise in Form einer logischen Abfrage, eines Zahlenwertvergleichs, einer Schnittmengenbildung oder dergleichen sowie vorzugsweise einer Kombination zumindest zweier derselben. Insbesondere ist denkbar, dass der Eignungsparameter in Abhängigkeit von einem bestimmten Satz von Patientenparametern und/oder in Abhängigkeit von einem bestimmten Satz von Rekrutierungsparametern maschinell ermittelt wird. Erfindungsgemäß ist der Eignungsparameter ein Gütemaß für eine Eignung des Patienten für die Studie, d.h. ein Match-Score. Es ist denkbar, dass der Eignungsparameter ein boolescher Parameter ist, beispielsweise mit den beiden Zuständen "Patient geeignet" und "Patient nicht geeignet". Erfindungsgemäß ist der Eignungsparameter ein Matchparameter, d.h. der Match-Score, der erfindungsgemäß mit einer Güte eines Matches zwischen dem Patienten und der Studie korreliert. Beispielsweise kann der Eignungsparameter ein Prozentwert einer Eignung sein. Ferner ist denkbar, dass der Eignungsparameter verschiedene diskrete Werte annehmen kann, beispielsweise Werte zwischen 0 und 5, zwischen 0 und 10, zwischen 0 und 100 oder beliebige andere diskrete Werte. Vorzugsweise wird der Eignungsparameter als eine Funktion mehrerer Patientenparameter und mehrerer Studienparameter bestimmt, wobei einzelne Parameter und/oder Matches unterschiedlich stark gewichtet werden können. Zudem ist denkbar, dass bestimmte Parameter als Ausschlussparameter gehandhabt werden. Beispielsweise ist denkbar, dass ein Patient als vollständig ungeeignet bewertet wird, wenn zumindest ein Patientenparameter nicht innerhalb eines gemäß einem entsprechenden Rekrutierungsparameter erlaubten Wertebereichs liegt und/oder nicht einem bestimmten Rekrutierungsparameter entspricht, wie beispielsweise einem Geschlecht und/oder einer bestimmten Medikation, aufgrund derer ein Patient beispielsweise ungeeignet für eine entsprechende Studie ist. Alternativ oder zusätzlich ist denkbar, dass der Eignungsparameter abhängig von einer Anzahl und/oder Ausprägung einer Übereinstimmung von Patientenparametern mit entsprechenden Rekrutierungsparametern ist.

Beispielsweise kann die Studie Rekrutierungsparameter enthalten, die ein bestimmtes Geschlecht, ein Alter in einem bestimmten Wertebereich, ein Vorhandensein einer bestimmten Erkrankung sowie einen Laborwert einer bestimmten gesundheitsrelevanten Größe in einem bestimmten Wertebereich von einem potentiell geeigneten Patienten fordern. Selbstverständlich sind beliebige andere dem Fachmann sinnvoll erscheinende Kombinationen denkbar. In diesem Fall könnte beispielsweise der Eignungsparameter einen mittleren Wert annehmen, wenn die Patientenparameter bezüglich Geschlecht und Vorhandensein der Erkrankung den Rekrutierungsparametern entsprechen und der Patient ein passendes Lebensalter aufweist, und einen hohen Wert annehmen, wenn beispielsweise zusätzlich ein Laborwert des Patienten in den entsprechenden Wertebereich fällt. Das Geschlecht könnte darüber hinaus beispielsweise als Ausschlusskriterium definiert sein, sodass beispielsweise bei einem Patienten mit einem Geschlecht, das nicht dem von der Studie geforderten entspricht, ein Eignungsparameter, der eine Nichteignung anzeigt, erstellt wird, unabhängig von einem Match der übrigen Parameter. Hierbei sind selbstverständlich ebenfalls beliebige dem Fachmann sinnvoll erscheinende Kombinationen und Strategien zur Ermittlung eines Eignungsparameters denkbar und die angeführten Beispiele sind vollständig nicht einschränkend zu verstehen.

Vorzugsweise umfasst die Zuordnung eine Verknüpfung des Patienten mit der Studie, insbesondere unter Vermerkung des Eignungsparameters. Einem bestimmten Patienten können vorzugsweise mehrere Studien sowie insbesondere entsprechende Eignungsparameter zugeordnet werden. Ebenso können einer bestimmten Studie mehrere Patienten sowie insbesondere entsprechende Eignungsparameter zugeordnet werden. Vorteilhaft umfasst das Verfahren zumindest einen Pseudonymisierungsschritt, in welchem zumindest ein Patientenparameter und/oder zumindest ein Patientendatensatz zumindest teilweise pseudonymisiert wird. Ebenso ist auch eine Anonymisierung denkbar. Grundsätzlich werden vorteilhaft sämtliche persönliche Daten des Patienten in dem Verfahren an geeigneter Stelle pseudonymisiert und/oder anonymisiert. Insbesondere findet in dem Zuordnungsschritt die Zuordnung zu dem Patienten in pseudonymisierter und/oder anonymisierter Form statt und/oder es wird die Zuordnungsinformation vorteilhaft in pseudonymisierter und/oder in anonymisierter Form auf dem Patientendatenserver hinterlegt.

Erfindungsgemäß wird in dem Zuordnungsschritt dem Patienten die Studie in Abhängigkeit von dem Eignungsparameter zugeordnet. Erfindungsgemäß wird dem Patienten die Studie zugeordnet, falls der Eignungsparameter eine Eignung des Patienten für die Studie anzeigt, indem der Eignungsparameter einen bestimmten Grenzwert über- oder unterschreitet. Dabei ist denkbar, dass der Grenzwert maschinell in Abhängigkeit von zumindest einer auf dem Patientenrekrutierungsserver und/oder auf dem Studiendatenserver hinterlegten Information angepasst wird. Beispielsweise kann der Grenzwert erhöht oder herabgesetzt werden, wenn sich eine Anzahl potentiell geeigneter Patienten erhöht oder reduziert. Zudem ist denkbar, dass der Grenzwert, beispielsweise von klinischem Personal und/oder von dem Patienten vorgebbar ist. Außerdem ist denkbar, dass der Grenzwert als ein Rekrutierungsparameter auf dem Studiendatenserver hinterlegt und/oder von einem Ersteller und/oder Verantwortlichen der Studie vorgebbar ist. Hierdurch kann vorteilhaft eine Eignungsinformation mit einem Patienten verknüpft werden. Vorteilhaft kann hierdurch ein einfaches und/oder effizientes Auffinden geeigneter Patienten erzielt werden.

Erfindungsgemäß werden in dem Zuordnungsschritt dem Patienten mehrere geeignete Studien zugeordnet. Insbesondere wird jeweils zumindest ein Studienparameter und vorteilhaft jeweils ein entsprechender Eignungsparameter dem Patienten zugeordnet und eine entsprechende Zuordnungsfunktion wird vorteilhaft auf dem Patientenrekrutierungsserver hinterlegt. Vorzugsweise wird für eine bestimmte Auswahl von Studien und/oder für alle Studien, zu denen Studiendaten auf dem Studiendatenserver hinterlegt sind, jeweils ein Eignungsparameter des Patienten erstellt und vorteilhaft werden geeignete Studien anschließend dem Patienten zugeordnet. Die Auswahl an Studien kann dabei maschinell und/oder auf der Grundlage von zumindest einem vorgebbaren Parameter erfolgen. Hierdurch kann vorteilhaft eine Zeit- und/oder Kostenersparnis bei einer Rekrutierung von Patienten für geeignete Studien erzielt werden.

Erfindungsgemäß wird in dem Zuordnungsschritt dem Patienten eine geordnete Liste geeigneter Studien zugeordnet, welche auf zumindest einem Server, insbesondere dem Patientenrekrutierungsserver, hinterlegt wird. Eine Studie ist dabei insbesondere geeignet, sofern der Patient als Proband für die Studie zumindest teilweise geeignet oder zumindest nicht ungeeignet ist. Zudem ist denkbar, dass eine weitere, insbesondere geordnete, Liste von aktuell ungeeigneten oder teilweise geeigneten Studien erstellt und dem Patienten zugeordnet wird, insbesondere eine Liste, die zukünftig potentiell geeignete Studien enthält. Hierbei ist eine automatisierte Entscheidung bezüglich einer künftigen potentiellen Eignung denkbar, beispielsweise auf Grundlage einer aktuellen Medikation und/oder einer gesundheitlichen Situation oder dergleichen eines Patienten, aufgrund derer der Patient aktuell für die Studie ungeeignet ist, deren Ende jedoch absehbar ist und/oder nach deren Ende der Patient für die Studie geeignet wäre. Hierdurch kann vorteilhaft ausgehend von einem bestimmten Patienten der Patient für eine Studie rekrutiert werden, wobei als Ausgangspunkt beispielsweise eine grundsätzliche Bereitschaft des Patienten, an einer Studie teilzunehmen, dient, ehe vorteilhaft eine geeignete Studie aus der Liste ausgewählt wird.

Erfindungsgemäß wird die geordnete Liste nach zumindest einem maschinell ermittelten und/oder nach zumindest einem vorgebbaren Kriterium geordnet maschinell erstellt. Vorteilhaft ist das Kriterium beispielsweise von dem Patienten und/oder von einem behandelnden Arzt und/oder von anderem klinischen Personal und/oder von einem Studienverantwortlichen vorgebbar. Erfindungsgemäß wird die Liste nach einem Gesamt-Match-Score geordnet. Alternativ oder zusätzlich ist denkbar, dass die Liste nach einem Datum, beispielsweise eines Studienbeginns und/oder einer Aufnahme des Patienten und/oder nach einem Zeitraum, beispielsweise einer Studiendauer, insbesondere grundsätzlich geeigneter Studien, und/oder einer Aufenthaltsdauer des Patienten oder dergleichen geordnet ist. Hierdurch kann vorteilhaft eine Wahrscheinlichkeit einer erfolgreichen Rekrutierung erhöht und/oder ein Aufwand bei einer Rekrutierung reduziert werden.

Des Weiteren wird erfindungsgemäß in zumindest einem Auswahlschritt eine Liste von abzugleichenden Studien, insbesondere in Abhängigkeit von zumindest einer dem Patienten und/oder der Studie zugeordneten Standortinformation, erstellt. Vorzugsweise werden in dem Abgleichschritt lediglich Studien herangezogen, die in dem Auswahlschritt der Liste abzugleichender Studien hinzugefügt wurden. Vorzugsweise erfolgt der Auswahlschritt vor dem Abgleichschritt. Die Standortinformation kann beispielsweise ein Ort einer Gesundheitsinstitution, an welcher sich der Patient aktuell aufhält und/oder welche der Patient beispielsweise für eine ambulante Behandlung vorzugsweise wiederholt aufsucht, und/oder ein Wohnort und/oder ein Ort eines Hausarztes und/oder ein Ort, an welchem die Studie durchgeführt wird, insbesondere ein Ort einer Gesundheitsinstitution, an welcher die Studie durchgeführt wird, und/oder ein Aufenthaltsort eines Studienverantwortlichen oder dergleichen sein. Vorteilhaft werden Studien der Liste von abzugleichenden Studien hinzugefügt, die an derselben Gesundheitsinstitution durchgeführt werden und/oder werden sollen, an welcher sich der Patient aktuell aufhält und/oder zukünftig, insbesondere voraussichtlich, aufhalten wird.

Beispielsweise ist denkbar, dass bei einer Registrierung des Patienten in der Gesundheitsinstitution, die insbesondere auch zumindest eine Information bezüglich einer voraussichtlichen Aufenthaltsdauer und/oder bezüglich einer Raumnummer des Patienten und dergleichen umfassen kann, verfügbare Studien, deren Studiendatensätze auf dem Studiendatenserver hinterlegt sind, nach Studien durchsucht wird, denen eine geeignete Standortinformation zugeordnet ist, insbesondere um die Liste von abzugleichenden Studien zu erstellen. Insbesondere in dem Fall, dass zumindest der Studiendatenserver als ein lokaler Server der Gesundheitsinstitution ausgebildet ist, ist denkbar, dass lediglich Studiendaten von Studien auf diesem hinterlegt und/oder hinterlegbar sind, die an der Gesundheitsinstitution durchgeführt werden. Es ist aber auch denkbar, dass Studiendaten zu Studien unterschiedlicher Gesundheitsinstitutionen auf dem Studiendatenserver hinterlegt und/oder hinterlegbar sind.

Ferner ist denkbar, dass die Liste von abzugleichenden Studien in Abhängigkeit von zumindest einem Datum und/oder einem Zeitraum und/oder einem Termin oder dergleichen erstellt wird. Beispielsweise könnte der Patient zur Teilnahme an einer bestimmten Studie ungeeignet sein, da dieser an bestimmten Terminen bereits einer anderen Studie zugeordnet ist und/oder einen Behandlungstermin hat oder dergleichen, wobei vorzugsweise die entsprechende Studie der Liste von abzugleichenden Studien nicht hinzuzufügen wäre. Ebenso ist denkbar, dass ein Patientenaufenthalt an der Gesundheitsinstitution voraussichtlich, insbesondere erheblich, kürzer ist als eine Studienlaufzeit und insbesondere deshalb für eine bestimmte Studie ungeeignet erscheint, die entsprechend vorteilhaft der Liste von abzugleichenden Studien nicht hinzuzufügen ist.

In einer vorteilhaften Ausgestaltung der Erfindung wird vorgeschlagen, dass der Auswahlschritt und/oder der Abgleichschritt und/oder der Zuordnungsschritt wiederholt durchgeführt werden. Erfindungsgemäß wird die Liste abzugleichender Studien abgearbeitet, um die geordnete Liste geeigneter Studien zu erstellen und dem Patienten zuzuordnen. Erfindungsgemäß wird die Liste abzugleichender Studien in Echtzeit wiederholt aktualisiert und/oder in Echtzeit wiederholt abgearbeitet. Vorteilhaft werden Patientenparameter mit Rekrutierungsparametern in Echtzeit abgeglichen und/oder Eignungsparameter in Echtzeit ermittelt. Erfindungsgemäß wird der Auswahlschritt, der Abgleichschritt und der Zuordnungsschritt automatisiert in bestimmten regelmäßigen und/oder vorgebbaren Zeitabständen wiederholt durchgeführt, beispielsweise täglich oder wöchentlich, beispielsweise um neu hinzugekommene Studien auf eine Eignung des Patienten für neu hinzugekommene Studien zu überprüfen. Zudem wird der Auswahlschritt und der Abgleichschritt und der Zuordnungsschritt erfindungsgemäß erneut durchgeführt, nachdem zusätzliche Patientenparameter, insbesondere von dem Patienten und/oder von klinischem Personal und/oder von einem Studienverantwortlichen, eingegeben worden sind. Hierdurch kann vorteilhaft eine hohe Aktualität erzielt werden. Zudem kann hierdurch über einen gesamten Zeitraum eines Aufenthalts eines Patienten an einer Gesundheitsinstitution eine hohe Wahrscheinlichkeit einer erfolgreichen Rekrutierung erzielt werden.

Erfindungsgemäß wird in zumindest einem Ausgabeschritt mittels zumindest einer Ein- und/oder Ausgabeeinheit zumindest eine Studieninformation ausgegeben. Bevorzugt ist die Ein- und/oder Ausgabeeinheit als eine Ein- und Ausgabeeinheit ausgebildet, die dazu vorgesehen ist, zumindest eine Information für einen Benutzer auszugeben und die zu einer Eingabe zumindest einer Information durch einen Benutzer, insbesondere denselben Benutzer, vorgesehen ist. Erfindungsgemäß wird eine Studieninformation einer Studie ausgegeben, für welche der Patient zumindest teilweise geeignet ist. Erfindungsgemäß ist die Ein- und/oder Ausgabeeinheit dazu vorgesehen, Studiendaten der Studien der geordneten Liste geeigneter Studien in geordneter Form auszugeben und darzustellen. Vorteilhaft ist die Ein- und/oder Ausgabeeinheit dazu vorgesehen, zu ausgegebenen Studien jeweils einen entsprechenden Eignungsparameter oder ein anhand dieser Eignungsparameter ermitteltes Eignungskriterium, beispielsweise in Form einer Punktzahl, einer Sterne-Bewertung, eines je nach Eignung mehr oder weniger gefüllten Balkens, eines Match-Scores oder dergleichen auszugeben. Des Weiteren ist die Ein- und/oder Ausgabeeinheit vorteilhaft dazu vorgesehen, die Studieninformationen in unterschiedlichen Detailstufen auszugeben, beispielsweise in Abhängigkeit von einer entsprechenden Auswahl und/oder Vorauswahl durch den Patienten und/oder das klinische Personal. Ferner ist denkbar, dass die Ein- und/oder Ausgabeeinheit zu einem Pushen von Studieninformation vorgesehen ist, beispielsweise in dem Fall, dass sich ein Eignungsparameter des Patienten für eine bestimmte Studie erhöht und/oder dass eine Studie mit einem hohen Match-Score neu hinzukommt oder dergleichen. Erfindungsgemäß ist die Ein- und/oder Ausgabeeinheit zu einer Anordnung in einem Patientenzimmer zumindest einer Gesundheitsinstitution vorgesehen. In einer nicht erfindungsgemäßen Alternative könnte die Ein- und/oder Ausgabeeinheit zu einer Anordnung in zumindest einem Ärztezimmer und/oder in zumindest einem Wartezimmer vorgesehen sein. Besonders bevorzugt ist die Ein- und/oder Ausgabeeinheit in zumindest einer Gesundheitsinstitution, insbesondere in zumindest einem Patientenzimmer und/oder in zumindest einem Ärztezimmer und/oder in zumindest einem Wartezimmer angeordnet und vorteilhaft, insbesondere permanent, installiert. Die Ein- und/oder Ausgabeeinheit kann eine Mehrzahl von Ein- und/oder Ausgabegeräten umfassen, von denen zumindest einige identisch oder unterschiedlich ausgebildet sein können. Beispielsweise kann die Ein- und/oder Ausgabeeinheit unterschiedliche Ein- und/oder Ausgabegeräte für Patientenzimmer, Ärztezimmer und/oder Wartezimmer umfassen. Hierdurch können vorteilhaft Studiendaten, insbesondere zu geeigneten Studien, in einfacher und/oder effizienter Weise bereitgestellt werden.

Erfindungsgemäß ist die Ein- und/oder Ausgabeeinheit Teil des Patientenrekrutierungssystems. Besonders bevorzugt ist die Ein- und/oder Ausgabeeinheit mit dem Patientenrekrutierungsserver verbindbar und/oder verbunden. Insbesondere ist die Ein- und/oder Ausgabeeinheit zur Eingabe zumindest eines Patientenparameters und/oder einer Teilnahmeabsicht und/oder zur Ausgabe zumindest einer Studieninformation vorgesehen. Die Studieninformation kann beispielsweise eine textuelle und/oder bildliche und/oder akustische und/oder audiovisuelle Beschreibung einer bestimmten Studie und/oder ein Ziel der Studie und/oder Teilnahmevoraussetzungen für die Studie und/oder eine Kontaktinformation zu einer verantwortlichen Person oder dergleichen umfassen. Vorzugsweise ist die Studieninformation dazu geeignet, dem Patienten zu ermöglichen, sich über eine bestimmte Studie zu informieren. Insbesondere ist zumindest ein zur Benutzung durch einen Patienten vorgesehenes Ein- und/oder Ausgabegerät der Ein- und/oder Ausgabeeinheit zur Eingabe des Patientenparameters vorgesehen. Der Patientenparameter kann insbesondere in diesem Fall beispielsweise eine patientenspezifische Information sein, die zu einer Ergänzung eines Patientendatensatzes geeignet, auf dem Patientendatenserver jedoch noch nicht abgelegt ist. Alternativ oder zusätzlich ist denkbar, dass der Patientenparameter mittels der Ein- und/oder Ausgabeeinheit von einem klinischen Personal, beispielsweise von einem behandelnden Arzt, eingegeben wird. Vorzugsweise ist mittels der Ein- und/oder Ausgabeeinheit, insbesondere durch den Patienten und/oder durch ein dem Patienten zugeordnetes klinisches Person, zumindest eine Information bezüglich einer Absicht und/oder Bereitschaft des Patienten, an einer bestimmten Studie teilzunehmen, als die Teilnahmeabsicht eingebbar. Hierdurch kann vorteilhaft eine Information bezüglich potentiell geeigneter Studien, insbesondere für einen bestimmten Patienten und oder für dessen behandelndes klinisches Personal, in effizienter und/oder umfassender Weise bereitgestellt werden.

Erfindungsgemäß umfasst die Ein- und/oder Ausgabeeinheit zumindest ein Display, welches in einem Patientenzimmer, insbesondere in dem Patientenzimmer des Patienten, angeordnet ist. Alternativ oder zusätzlich ist eine Anordnung in einem Ärztezimmer und/oder in einem Wartezimmer denkbar. Insbesondere kann die Ein- und/oder Ausgabeeinheit eine Mehrzahl von Displays aufweisen, über welche Informationen von Patienten und/oder von klinischem Personal eingebbar und/oder abrufbar sind. Besonders bevorzugt ist das Display zumindest ein Teil eines dem Patienten zugeordneten Ein- und/oder Ausgabegeräts. Vorzugsweise ist das Display in dem Patientenzimmer installiert. Es ist aber auch denkbar, dass das Display und/oder das dem Patienten zugeordnete Ein- und/oder Ausgabegerät entfernbar ausgebildet ist, beispielsweise nach Art eines tragbaren Displays und/oder Computers. Vorzugsweise ist zumindest eine Standortinformation des dem Patienten zugeordneten Ein- und/oder Ausgabegeräts auf dem Patientendatenserver hinterlegt, beispielsweise in Form von Koordinaten und/oder in Form einer Zimmernummer und/oder einer Stockwerksinformation oder dergleichen.

Zudem ist denkbar, dass die Ein- und/oder Ausgabeeinheit zumindest eine mobile Vorrichtung umfasst. Beispielsweise kann die mobile Vorrichtung dem Patienten und/oder dem klinischen Personal, insbesondere dem behandelnden Arzt, zugeordnet sein und/oder gehören. Die mobile Vorrichtung kann insbesondere als ein Smartphone, ein Tablet-PC, eine Datenbrille, eine Smartwatch, ein Smartband, ein Smart Cloth, ein Smart Ring, ein beliebiges anderes Wearable oder dergleichen ausgebildet sein. Vorzugsweise ist die mobile Vorrichtung zu einer Verbindung zumindest mit dem Patientenrekrutierungsserver vorgesehen. Insbesondere bildet die mobile Vorrichtung zumindest ein Ein- und/oder Ausgabegerät der Ein- und/oder Ausgabeeinheit aus. Hierdurch kann vorteilhaft ein hoher Benutzerkomfort erzielt werden. Zudem kann eine hohe Kosteneffizienz erzielt werden, wenn beispielsweise ohnehin vorhandene mobile Geräte in das Patientenrekrutierungssystem integriert werden.

In einer vorteilhaften Ausgestaltung der Erfindung wird vorgeschlagen, dass mittels der Ein- und/oder Ausgabeeinheit zumindest ein Teilnahmeabsichtsparameter des Patienten eingegeben und auf zumindest einem Server, insbesondere auf dem Patientenrekrutierungsserver und/oder auf dem Patientendatenserver, hinterlegt wird. Der Teilnahmeabsichtsparameter kann ein boolescher Parameter sein, der beispielsweise den Wert "Teilnahme beabsichtigt" annehmen kann. Es ist denkbar, dass eine Information bezüglich einer nicht beabsichtigten Teilnahme des Patienten automatisiert und/oder als Standardwert auf dem Patientenrekrutierungsserver hinterlegt wird, insbesondere für alle Studien der geordneten Liste geeigneter Studien, und diese Information vorzugsweise für eine bestimmte Studie gelöscht wird, sofern zu dieser bestimmten Studie ein entsprechender Teilnahmeabsichtsparameter eingegeben wird. Es ist auch denkbar, dass der Teilnahmeabsichtsparameter eine Wertung enthält. Beispielsweise kann zu wenigstens einigen Studien der geordneten Liste geeigneter Studien eine Präferenz des Patienten hinterlegt werden, die insbesondere nach einem Interesse des Patienten an einer Teilnahme geordnet ist. Insbesondere kann der Teilnahmeabsichtsparameter, vorteilhaft wahlweise, von dem Patienten und/oder von dem klinischen Personal eingegeben werden. Zudem ist denkbar, dass mittels der Ein- und/oder Ausgabeeinheit eine Information bezüglich einer Teilnahmeabsicht und/oder bezüglich eines grundsätzlichen Interesses des Patienten für ein klinisches Personal zur Verfügung gestellt wird. Beispielsweise kann ein behandelnder Arzt über ein grundsätzliches Interesse eines Patienten an einer bestimmten Studie informiert werden und vorzugsweise anschließend Kontakt mit dem Patienten bezüglich der Studie aufnehmen und/oder einen Studienverantwortlichen informieren. Hierdurch kann vorteilhaft eine Wahrscheinlichkeit einer erfolgreichen Rekrutierung erhöht werden.

Außerdem wird vorgeschlagen, dass zumindest ein Ausgabeparameter für die Ausgabe der Studieninformation in Abhängigkeit von zumindest einem Patientenmerkmal, insbesondere von zumindest einem Patientenparameter, vorzugsweise maschinell, angepasst wird. Das Patientenmerkmal kann beispielsweise ein Alter und/oder ein Geschlecht und/oder ein Bildungsstand und/oder eine Krankheitsgeschichte und/oder eine persönliche Präferenz und/oder ein beliebiger geeigneter Patientenparameter sein. Insbesondere ist das Patientenrekrutierungssystem und/oder die Ein- und/oder Ausgabeeinheit zu einer kontextsensitiven Darstellung von Informationen, insbesondere der Studieninformationen, vorgesehen. Vorzugsweise ist der Ausgabeparameter ein Darstellungsparameter, insbesondere des Displays. Beispielsweise wird eine Schriftgröße und/oder eine Displayhelligkeit und/oder eine Sprache und/oder eine Ausführlichkeit und/oder Formulierung von Inhalten und/oder eine Lautstärke und/oder eine Anordnung von Teilinformationen auf einem Informationsbereich, beispielsweise auf einem Display, oder dergleichen, insbesondere automatisiert, angepasst. Zudem ist denkbar, dass Fachtermini, beispielsweise in Studienbeschreibungen, durch patientengerechte Sprache und/oder durch geläufige Begriffe automatisiert ersetzt werden, insbesondere in Abhängigkeit von zumindest einem Patientenparameter wie beispielsweise einer Präferenz des Patienten. Beispielsweise könnte wahlweise der Begriff "Pneumonie" durch den Begriff "Lungenentzündung" ersetzt werden oder dergleichen. Zudem ist denkbar, dass mittels zumindest einer automatisierten semantischen Analyse einer Studienbeschreibung zumindest eine vereinfachte und/oder leichter verständliche und/oder gekürzte Studienbeschreibung erstellt wird. Ferner ist denkbar, dass das Patientenmerkmal automatisiert erfasst wird, beispielsweise mittels zumindest einer Abfrage zumindest eines Patientenparameters vom Patientendatenserver und/oder mittels zumindest einer Abfrage einer beispielsweise auf einem externen Server hinterlegten Information oder dergleichen. Es ist auch denkbar, dass die Ein- und/oder Ausgabeeinheit zu einer Eingabe zumindest eines Zielausgabeparameters durch den Patienten und/oder durch das behandelnde klinische Personal vorgesehen ist, der den Ausgabeparameter festlegt. Hierdurch kann vorteilhaft eine gezielt auf einen Patienten abgestimmte Darstellung von Information erzielt und insbesondere der Patient zu einer Teilnahme an einer Studie animiert werden.

Zudem wird vorgeschlagen, dass zumindest eine hierarchische Bewertungsliste mit mehreren Bewertungskriterien anhand mehrerer Rekrutierungsparameter der Studie maschinell und/oder automatisiert erstellt wird, anhand derer der Eignungsparameter ermittelt wird. Insbesondere werden die Bewertungskriterien maschinell aus den Rekrutierungsparametern ermittelt. Selbstverständlich ist auch denkbar, dass die Bewertungsliste zumindest teilweise manuell erstellt und/oder vorgebbar ist, beispielsweise von einem Studienverantwortlichen. Vorzugsweise umfasst die Bewertungsliste eine hierarchisch aufgebaute logische Vergleichsvorschrift zum Vergleich der Rekrutierungsparameter mit den Patientenparametern. Wie oben beschrieben können dabei Bewertungskriterien Einschluss- und/oder Ausschlusskriterien sein. Zudem ist denkbar, dass Bewertungskriterien mehrere unterschiedliche, insbesondere kontinuierlich verteilte, Werte erzeugen können, beispielsweise Wahrscheinlichkeiten und/oder Punktzahlen und/oder Teil-Match-Scores oder dergleichen. Vorzugsweise wird in dem Bewertungsschritt anhand der Bewertungsliste als Eignungsparameter ein Match-Score, insbesondere in Abhängigkeit von mehreren Teil-Match-Scores, und/oder eine Anzahl an Matches zwischen Rekrutierungsparametern und Patientenparametern ermittelt. Es ist denkbar, dass die Bewertungsliste und/oder entsprechende Rekrutierungsparameter anhand einer textuellen Beschreibung einer Studie zumindest teilweise automatisiert erstellt wird/werden. Hierdurch können vorteilhaft auch komplexe Voraussetzungen, die zu einer Eignung für eine Studie erfüllt sein müssen, effizient ermittelt und/oder überprüft werden.

In einer vorteilhaften Ausgestaltung der Erfindung wird vorgeschlagen, dass den Bewertungskriterien jeweils zumindest ein Gewichtungsparameter, insbesondere maschinell und/oder automatisiert, zugeordnet wird. Es ist denkbar, dass die Gewichtungsparameter zumindest teilweise automatisiert erstellt werden, beispielsweise auch in Form von automatisiert erstellten Startwerten, die insbesondere nachträglich überschrieben werden können. Beispielsweise können zumindest einige der Gewichtungsparameter von einem Studienverantwortlichen und/oder einem Auftraggeber und/oder einem einer entsprechenden Studie zugeordneten klinischen Personal oder dergleichen anpassbar sein, insbesondere mittels der Ein- und/oder Ausgabeeinheit. Beispielsweise kann ein Gewichtungsparameter eines bestimmten Bewertungskriteriums geändert werden, um eine größere oder eine kleinere Anzahl von Patienten als potentiell geeignet zu erkennen und/oder um Anpassungen in der Rekrutierung beispielsweise an neue Erkenntnisse und/oder Studienteilergebnisse vorzunehmen. Ferner ist denkbar, dass zumindest ein Gewichtungsparameter maschinell anhand zumindest einer Studieninformation ermittelt wird, beispielsweise mittels zumindest einer semantischen Analyse einer textuellen Beschreibung und/oder einer Analyse eines Studienziels oder dergleichen. Hierdurch kann vorteilhaft eine Güte einer Identifikation geeigneter Patienten verbessert werden.

Ferner wird vorgeschlagen, dass zumindest eine Liste von Bewertungskriterien, die zumindest eine Eingabe zumindest eines klinischen Personals erfordern, erstellt wird. Beispielsweise kann es sich hierbei um eine Liste von automatisiert erstellten Bewertungskriterien handeln, die dem klinischen Personal zu einer Überprüfung und/oder zu einer Freigabe bereitgestellt wird. Ferner ist denkbar, dass es sich um eine Liste von Bewertungskriterien handelt, die nicht vollständig automatisiert der Liste von Bewertungskriterien hinzugefügt wurden und/oder werden können und/oder die nicht vollständig automatisiert fertiggestellt wurden und/oder werden können. Alternativ oder zusätzlich ist denkbar, dass die Liste von Bewertungskriterien, insbesondere mittels der Ein- und/oder Ausgabeeinheit, von einem Studienverantwortlichen und/oder von einem klinischen Personal überprüft und/oder freigegeben und/oder editiert und/oder ergänzt und/oder umorganisiert wird. Hierdurch kann vorteilhaft eine hohe Flexibilität hinsichtlich einer Formulierung von Bewertungskriterien und/oder -strategien erzielt werden.

Vorteilhafte Eigenschaften hinsichtlich einer Bereitstellung von Information für einen an einer Studie interessierten Patienten können insbesondere erzielt werden, wenn zumindest eine Kontaktinformation zu der Studie für den Patienten bereitgestellt wird. Insbesondere werden Kontaktinformationen zu Studien bereitgestellt, zu denen zumindest eine Teilnahmeabsicht des Patienten vorliegt. Ferner werden vorteilhaft Kontaktinformationen zu Studien bereitgestellt, zu denen der Patient und/oder das klinische Person, insbesondere mittels der Ein- und/oder Ausgabeeinheit, zumindest eine Information bezüglich eines Interesses an der Studie eingegeben hat. Beispielsweise werden dem Patienten Informationen zu den Studien der geordneten Liste geeigneter Studien bereitgestellt, die der Patient, beispielsweise zu einer näheren Betrachtung, insbesondere mittels zumindest einer Eingabe einer Auswahl mittels der Ein- und/oder Ausgabeeinheit entscheidet. Es ist denkbar, dass die Ein- und/oder Ausgabeeinheit dazu vorgesehen ist, zusätzlich einen Kontakt zu einer Kontaktperson der Studie herzustellen, beispielsweise in Form eines Anrufs und/oder in Form einer Benachrichtigung oder dergleichen.

Eine hohe Zuverlässigkeit hinsichtlich einer Ermittlung einer Eignung und/oder hinsichtlich einer Rekrutierung geeigneter Patienten kann insbesondere erzielt werden, wenn in zumindest einem Eingabeschritt mittels zumindest einer Ein- und/oder Ausgabeeinheit zumindest ein weiterer Patientenparameter abgefragt und/oder eingegeben wird. Insbesondere wird der weitere Patientenparameter von dem Patienten und/oder von dem klinischen Personal erfragt. Vorzugsweise wird eine Art des weiteren Patientenparameters automatisiert ermittelt, beispielsweise im Fall eines teilweisen Matches eines Patientendatensatzes mit einem Studiendatensatz, insbesondere zur vollständigen oder zumindest vollständigeren Ermittlung eines Eignungsparameters. Der weitere Patientenparameter kann dabei ein beliebiger Patientenparameter sein, der insbesondere noch nicht auf dem Patientendatenserver hinterlegt ist und/oder vorteilhaft nach dessen Eingabe auf dem Patientendatenserver hinterlegt wird.

Ferner wird vorgeschlagen, dass mittels der Ein- und/oder Ausgabeeinheit, insbesondere mittels eines dem klinischen Personal zugeordneten Ein- und/oder Ausgabegeräts, zumindest eine Eignungsinformation des Patienten für ein dem Patienten zugeordnetes klinisches Personal zur Verfügung gestellt wird. Insbesondere ist die Ein- und/oder Ausgabeeinheit dazu vorgesehen, die geordnete Liste von Studien sowie den Studien zugeordnete Eignungskriterien und/oder Eignungsparameter zur Verfügung zu stellen, insbesondere auszugeben und/oder anzuzeigen. Vorzugsweise ist die Ein- und/oder Ausgabeeinheit dazu vorgesehen, eine Auswahl unterschiedlicher Patienten durch das klinische Personal zu verarbeiten und bevorzugt eine entsprechende, einem jeweils ausgewählten Patienten zugeordnete, Eignungsinformation sowie besonders vorteilhaft zugehörige Studieninformationen zur Verfügung zu stellen, beispielsweise bei einer Visite und/oder bei einer Durchsicht einer Belegung oder dergleichen. Zudem ist denkbar, dass die Ein- und/oder Ausgabeeinheit dazu vorgesehen ist, dem Patienten zumindest eine Studieninformation in Abhängigkeit von zumindest einer Vorauswahl durch ein klinisches Personal bereitzustellen und/oder auszugeben, wobei die Vorauswahl vorzugsweise nach einer Ausgabe der Eignungsinformation des Patienten für das klinische Personal von dem klinischen Personal eingegeben werden kann. Hierdurch kann vorteilhaft eine hohe Kosteneffizienz und/oder eine Zeitersparnis erzielt werden, insbesondere da klinisches Personal potentiell geeignete Patienten gezielt zur Teilnahme an klinischen Studien animieren kann.

Erfindungsgemäß wird zumindest ein Patientenparameter mittels zumindest einer Sensoreinheit, insbesondere einer Sensoreinheit der Ein- und/oder Ausgabeeinheit, automatisiert erfasst und maschinell verarbeitet. Erfindungsgemäß weist das Patientenrekrutierungssystem die Sensoreinheit auf. Die Sensoreinheit kann beispielsweise in einem Patientenzimmer und/oder an einem Patientenbett, insbesondere permanent, installiert sein. Es ist auch denkbar, dass die Sensoreinheit als eine mobile Sensoreinheit ausgebildet ist, die insbesondere an unterschiedlichen Positionen und/oder an unterschiedlichen Patientenbetten und/oder in unterschiedlichen Patientenzimmern anbringbar sein kann. Zudem ist denkbar, dass die Sensoreinheit an einen Körper des Patienten anbringbar ist, beispielsweise in Form eines Bands und/oder eines Gürtels und/oder einer Kopfbedeckung und/oder eines Kontaktpunkts oder dergleichen. Die Sensoreinheit kann auch zumindest einen Teil eines Kleidungsstücks des Patienten umfassen. Es ist auch denkbar, dass die Sensoreinheit zumindest ein Teil der mobilen Vorrichtung ist. Die Sensoreinheit kann einen Körpertemperatursensor und/oder eine Bilderfassungseinheit und/oder einen Gewichtssensor und/oder einen Körperparametersensor und/oder einen Fingerabdruckscanner oder dergleichen umfassen. Beispielsweise kann die Sensoreinheit eine Kamera und/oder einen Bewegungssensor und/oder einen anderen Sensor, beispielsweise eines Smartphones und/oder einer Datenbrille und/oder eines Wearables oder auch eines an dem Patientenbett und/oder in dem Patientenzimmer angeordneten, insbesondere fest installierten, Ein- und/oder Ausgabegeräts oder dergleichen umfassen. Vorzugsweise ist die Sensoreinheit dazu vorgesehen, zumindest einen erfassten Parameter und/oder zumindest eine Statusinformation und/oder beliebige Daten an zumindest einen Server, insbesondere an den Patientendatenserver und/oder an den Studiendatenserver und/oder an den Patientenrekrutierungsserver zu übermitteln. Die Sensoreinheit kann beispielsweise zur Ermittlung eines Körpergewichts und/oder einer Körpertemperatur und/oder eines Fingerabdrucks und/oder eines Schlafverhaltens und/oder einer Müdigkeit und/oder beliebiger anderer Patientenparameter vorgesehen sein. Zudem ist denkbar, dass die Sensoreinheit zu einer Bilderfassung vorgesehen ist. Insbesondere in diesem Fall ist denkbar, dass der Patientenrekrutierungsserver und/oder die Ein- und/oder Ausgabeeinheit und/oder die Sensoreinheit zu einer Bilderkennung vorgesehen ist, anhand derer insbesondere zumindest ein Patientenparameter ermittelbar ist. Beispielsweise kann mittels der Sensoreinheit zumindest ein Bild, beispielsweise des Patienten und/oder einer Medikamentenverpackung und/oder einer Wunde und/oder eines Körperteils und/oder eines Verbands des Patienten oder dergleichen erfasst werden, anhand dessen zumindest ein Patientenparameter ermittelbar ist. Insbesondere ist denkbar, dass das Verfahren einen Verfahrensschritt zur Ermittlung einer Medikamenten-Benutzungsinformation des Patienten aufweist, indem der Patient beispielsweise zu einem Einscannen und/oder Abfotografieren von Rezepten und/oder Medikamentenpackungen und/oder Tabletten oder dergleichen aufgefordert wird und/oder in welchem Verfahrensschritt der Patient entsprechende Bilder aufnimmt und/oder Scans durchführt und/oder in welchem Verfahrensschritt vorteilhaft entsprechend erfasste Bilder und/oder Scans maschinell analysiert werden, um die entsprechenden Patientenparameter automatisiert zu erfassen und/oder auf dem Patientendatenserver zu hinterlegen.

Ein hoher Grad an Bedienkomfort und/oder eine wirkungsvolle Rekrutierung aufgrund einer patientenspezifischen Darstellung können insbesondere erzielt werden, wenn zumindest ein Ausgabeparameter für eine Ausgabe zumindest einer Studieninformation in Abhängigkeit von dem mittels der Sensoreinheit erfassten Patientenparameter angepasst wird. Beispielsweise ist denkbar, dass der Ausgabeparameter in Abhängigkeit von einem erkannten Alter und/oder einer erkannten Beeinträchtigung beispielsweise eines Sehvermögens des Patienten und/oder in Abhängigkeit von mittels der Sensoreinheit erkannten Medikamenten, die dem Patienten aktuell verschrieben sind, und/oder einer erkannten Müdigkeit des Patienten oder dergleichen angepasst wird. Erfindungsgemäß ist die Sensoreinheit dazu vorgesehen, zumindest ein Einmaligkeitsmerkmal eines Patienten zu erkennen, wie beispielsweise einen Fingerabdruck, ein Gesicht, ein Auge oder auch beispielsweise einen RFID-Chip oder dergleichen, den der Patient am Körper trägt und/oder der in ein, insbesondere von der Gesundheitsinstitution zur Verfügung gestelltes, Kleidungsstück des Patienten integriert ist. Erfindungsgemäß wird alternativ oder zusätzlich ein nicht zwingend eindeutiges aber dem Patienten zugeordnetes Merkmal, wie beispielsweise ein Körpergewicht, ein Geschlecht, ein geschätztes Alter, eine Augenfarbe und/oder dergleichen, insbesondere auch in Kombination, hinterlegt und/oder mit aktuellen Erfassungen der Sensoreinheit verglichen, um einen Patienten zu erkennen. Beispielsweise kann erkannt werden, falls ein anderer als ein gemäß einem Belegungsplan vorgesehener Patient in einem bestimmten Patientenbett liegt und/oder ein bestimmtes Ein- und/oder Ausgabegerät benutzt und/oder bestimmte, für den korrekten Patienten vorgesehene Studieninformationen abfragen möchte oder dergleichen. Erfindungsgemäß wird ein Display und/oder ein anderes Ausgabemittel der Ein- und/oder Ausgabeeinheit zumindest vorübergehend deaktiviert und/oder gesperrt, wenn ein von dem Patienten abweichender anderer Patient und/oder kein Patient erkannt wird. Vorteilhaft kann hierdurch eine Anzeige sensibler und/oder persönlicher Daten für nicht autorisierte Personen vermieden werden.

Vorteilhafte Eigenschaften hinsichtlich eines Bedienkomforts und/oder einer effizienten Patientenrekrutierung können insbesondere mit einer Ein- und/oder Ausgabeeinheit erzielt werden, die zu einer Verwendung in einem erfindungsgemäßen Patientenrekrutierungssystem vorgesehen ist.

Die Erfindung betrifft zudem eine Verwendung des erfindungsgemäßen Verfahrens und/oder des erfindungsgemäßen Patientenrekrutierungssystems zur Rekrutierung von zumindest einem Patienten für zumindest eine Studie, insbesondere für eine klinische Studie, vorteilhaft für eine klinische Studie der Phase III, die beispielsweise ein Testen eines therapeutischen Effekts eines Wirkstoffs sowie insbesondere dessen Zulassung zum Ziel hat. Selbstverständlich ist auch eine Verwendung zur Rekrutierung von Patienten für eine beliebige andere, insbesondere klinische, Studie denkbar.

Das erfindungsgemäße Verfahren beziehungsweise das erfindungsgemäße Patientenrekrutierungssystem sollen hierbei nicht auf die oben beschriebenen Anwendungen und Ausführungsformen beschränkt sein. insbesondere können das erfindungsgemäße Verfahren beziehungsweise das erfindungsgemäße Patientenrekrutierungssystem zu einer Erfüllung einer hierin beschriebenen Funktionsweise eine von einer hierin genannten Anzahl von einzelnen Elementen und/oder Bauteilen und/oder Einheiten und/oder Verfahrensschritten abweichende Anzahl aufweisen. Insbesondere kann das erfindungsgemäße Verfahren spezielle Verfahrensschritte beinhalten, in welchen jeweils zumindest eines der oben beschriebenen Merkmale des erfindungsgemäßen Patientenrekrutierungssystems beziehungsweise eine damit verbundene Funktionalität zumindest teilweise implementiert wird. Zudem sollen bei den in dieser Offenbarung angegebenen Wertebereichen auch innerhalb der genannten Grenzen liegende Werte als offenbart und als beliebig einsetzbar gelten. Sofern nicht anders angegeben, ist weiterhin für sämtliche beschriebene Verfahrensschritte und/oder Vorgänge denkbar, dass diese zumindest teilweise und vorteilhaft vollständig maschinell und/oder automatisiert ausgeführt werden und/oder das Patientenrekrutierungssystem zu einer zumindest teilweise und vorteilhaft vollständig maschinellen und/oder automatisierten Ausführung derselben vorgesehen ist und/oder einen entsprechenden Prozess umfasst und/oder zu dessen Ausführung vorgesehen ist. Zudem kann "maschinell" im Rahmen dieser Offenbarung insbesondere auch "automatisiert" umfassen, wobei selbstverständlich auch maschinelle nicht automatisierte Vorgänge denkbar sind.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Gesundheitsinstitution mit einem Patientenrekrutierungssystem in einer schematischen Darstellung,
- Fig. 2: ein schematisches Ablaufdiagramm eines Verfahrens zur Patientenrekrutierung,
- Fig. 3: eine hierarchische Bewertungsliste in einer schematischen Darstellung und
- Fig. 4: ein Ein- und Ausgabegerät einer Ein- und/oder Ausgabeeinheit des Patientenrekrutierungssystems in einer schematischen Darstellung.

### Beschreibung der Ausführungsbeispiele

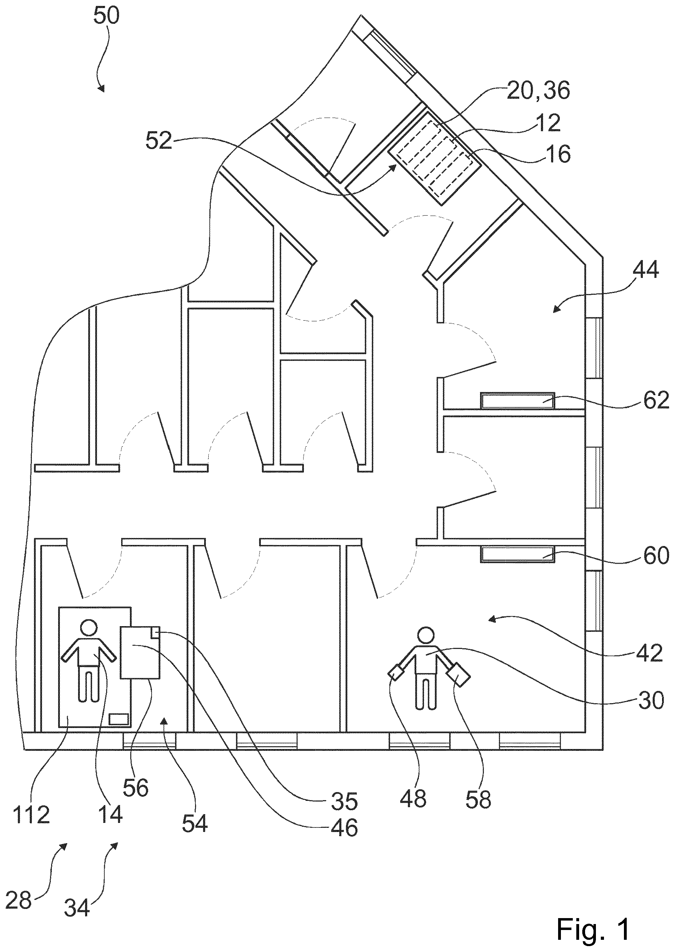

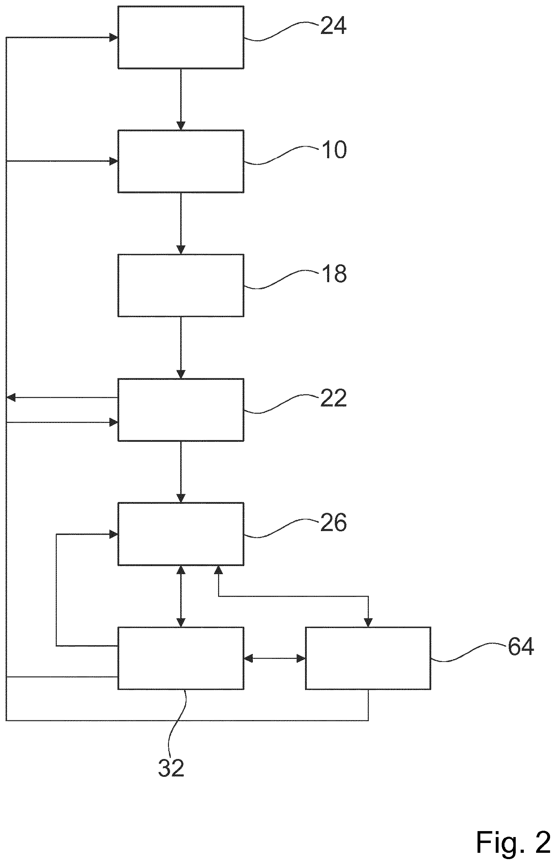

Figur 1 zeigt eine Gesundheitsinstitution 50 mit einem Patientenrekrutierungssystem 34 in einer schematischen Darstellung. Die Gesundheitsinstitution 50 kann beispielsweise eine Arztpraxis, ein Ärztehaus oder vorzugsweise ein Krankenhaus oder dergleichen sein. Das Patientenrekrutierungssystem 34 ist im vorliegenden Fall zu einer Durchführung eines Verfahrens zur Patientenrekrutierung für eine klinische Studie vorgesehen. Ein schematisches Ablaufdiagramm des Verfahrens zur Patientenrekrutierung ist in der Figur 2 dargestellt. Das Patientenrekrutierungssystem 34 und/oder das dargestellte Verfahren zur Patientenrekrutierung können zur Rekrutierung von zumindest einem Patienten 14 für zumindest eine Studie verwendet werden, insbesondere für eine klinische Studie, vorteilhaft für eine klinische Studie der Phase III, die beispielsweise ein Testen eines therapeutischen Effekts eines Wirkstoffs sowie insbesondere dessen Zulassung zum Ziel hat.

Das Patientenrekrutierungssystem 34 weist zumindest einen Patientenrekrutierungsserver 36 auf. Erfindungsgemäß weist das Patientenrekrutierungssystem 34 ferner zumindest einen Patientendatenserver 12 sowie zumindest einen Studiendatenserver 16 auf. Der Patientendatenserver 12, der Studiendatenserver 16 und der Patientenrekrutierungsserver 36 sind im vorliegenden Fall jeweils Teil eines gemeinsamen dedizierten Servers 52, der in ein Intranet der Gesundheitsinstitution 50 eingebunden ist. Insbesondere sind der Patientendatenserver 12, der Studiendatenserver 16 und der Patientenrekrutierungsserver 36 miteinander verbunden und/oder in einem gemeinsamen Netzwerk angeordnet. Es ist aber selbstverständlich denkbar, dass der Patientendatenserver 12, der Studiendatenserver 16 und/oder der Patientenrekrutierungsserver 36 zumindest teilweise getrennt voneinander und/oder zumindest teilweise außerhalb der Gesundheitsinstitution 50 und/oder deren Intranet und/oder als eine Cloud oder dergleichen ausgebildet sind.

Im dargestellten Fall hält sich ein Patient 14 in einem Patientenzimmer 54 der Gesundheitsinstitution 50 auf. Der Patient 14 kann beispielsweise stationär behandelt werden und/oder worden sein. Dem Patienten 14 ist ein klinisches Personal 30 zugeordnet, beispielsweise ein behandelnder Arzt. Das klinische Personal 30 kann dabei insbesondere von mehreren Personen, beispielsweise Ärzten und/oder Krankenpflegern und/oder Physiotherapeuten oder dergleichen gebildet werden, wie etwa in einem Schichtbetrieb oder dergleichen.

Im vorliegenden Fall existiert eine Mehrzahl klinischer Studien, die beispielsweise unmittelbar an der Gesundheitsinstitution 50 aktuell und/oder zukünftig durchgeführt werden. Im vorliegenden Fall sind Studiendatensätze zu diesen Studien auf dem Studiendatenserver 16 hinterlegt. Vorzugsweise sind auf dem Studiendatenserver 16 lediglich Studiendaten zu Studien der Gesundheitsinstitution 50 hinterlegt. Es ist aber ebenso denkbar, dass alternativ oder zusätzlich Studiendaten zu Studien hinterlegt sind, die an anderen Orten und/oder Gesundheitsinstitutionen aktuell und/oder zukünftig durchgeführt werden. Studiendaten und insbesondere Studiendatensätze können beispielsweise von Studienverantwortlichen erstellt und/oder auf dem Studiendatenserver 16 hinterlegt werden und/oder worden sein. Eine Erstellung von Studiendaten kann dabei zumindest teilweise automatisiert erfolgen, beispielsweise basierend auf Eingaben in eine entsprechende Eingabemaske. Insbesondere können auch unvollständige Studiendatensätze auf dem Studiendatenserver 16 hinterlegbar und/oder hinterlegt sein.

Zudem existiert im vorliegenden Fall eine Mehrzahl von Patientendatensätzen, die auf dem Patientendatenserver 12 hinterlegt sind. Patientendatensätze können dabei sowohl ehemaligen als auch aktuellen und/oder zukünftigen Patienten zugeordnet sein. Vorzugsweise enthält ein bestimmter Patientendatensatz zumindest Teile einer Behandlungsvergangenheit des jeweiligen Patienten 14, dessen Geschlecht, dessen Alter, dessen Medikation und/oder beliebige andere Patientendaten. Patientendaten können beispielsweise auf Eingaben und/oder Angaben von Patienten, von klinischem Personal, von einer Krankenkasse eines entsprechenden Patienten und dergleichen mehr basieren und/oder zumindest teilweise automatisiert erstellt werden und/oder worden sein. insbesondere können auch unvollständige Patientendatensätze auf dem Patientendatenserver 12 hinterlegt und/oder hinterlegbar sein.

Zudem existiert im vorliegenden Fall eine Mehrzahl von Rekrutierungsdatensätzen, die auf dem Patientenrekrutierungsserver 36 hinterlegt sind. Ein Rekrutierungsdatensatz enthält hierbei vorteilhaft zumindest einen Rekrutierungsparameter. insbesondere enthält ein Rekrutierungsdatensatz einer bestimmten Studie Angaben bezüglich bestimmter Merkmale, die ein Patient 14 aufweisen sollte und/oder muss, um für eine Teilnahme an der bestimmten Studie geeignet zu sein. Beispielsweise können ein Alter von Probanden, deren Medikation, deren Geschlecht, deren Krankheitsgeschichte, deren Gewicht und dergleichen mehr entsprechende Rekrutierungsparameter bilden. Vorzugsweise sind Rekrutierungsparameter von einem Studienverantwortlichen eingebbar. Es ist auch denkbar, dass Rekrutierungsparameter automatisiert, insbesondere anhand zumindest einer Eingabe, erstellt werden. Hierfür ist eine beliebige Art und Weise einer Formulierung derartiger Parameter denkbar, beispielsweise als logische Verknüpfung, als Eingabe in einer geeigneten Eingabemaske oder dergleichen. Zudem ist eine automatisierte Formulierung von Rekrutierungsparametern beispielsweise anhand zumindest einer semantischen Analyse zumindest einer Studienbeschreibung und/oder eines Anforderungsprofils denkbar. Grundsätzlich können auch unvollständige Rekrutierungsdatensätze auf dem Patientenrekrutierungsserver 36 hinterlegbar und/oder hinterlegt sein.

Erfindungsgemäß werden Patientendaten grundsätzlich in anonymisierter und/oder pseudonymisierter Form zwischen dem Patientendatenserver 12, dem Studiendatenserver 16 und dem Patientenrekrutierungsserver 36 ausgetauscht.

Der Patientenrekrutierungsserver 36 ist zu einer Ausführung zumindest eines Abgleichprozesses zum Abgleich zumindest eines Patientenparameters zumindest eines Patienten 14 mit zumindest einem Rekrutierungsparameter zumindest einer Studie vorgesehen. Ferner ist der Patientenrekrutierungsserver 36 zu einer Ausführung zumindest eines Bewertungsprozesses zur Generierung zumindest eines Eignungsparameters einer Eignung des Patienten 14 für die Studie in Abhängigkeit von dem Patientenparameter und dem Rekrutierungsparameter vorgesehen. Zudem ist der Patientenrekrutierungsserver 36 zur Ausführung zumindest eines Zuordnungsprozesses zur Erzeugung zumindest einer Zuordnung zwischen der Studie und dem Patienten 14 in Abhängigkeit von dem Eignungsparameter vorgesehen.

Wie oben erwähnt, wird mittels des Patientenrekrutierungssystems 34 ein Verfahren zur Patientenrekrutierung für klinische Studien durchgeführt, dessen Ablauf schematisch in der Figur 2 dargestellt ist. Die Darstellung von Verfahrensschritten und/oder Abläufen des Verfahrens ist hierbei rein exemplarisch und insbesondere nicht abschließend zu verstehen. Zudem können beliebige Verfahrensschritte wiederholt und/oder in einer anderen als der dargestellten Reihenfolge ausgeführt werden.

Das Verfahren zur Patientenrekrutierung umfasst zumindest einen Abgleichschritt 10, in welchem zumindest ein auf dem Patientendatenserver 12 hinterlegter Patientenparameter zumindest eines Patienten 14 mit zumindest einem auf dem Studiendatenserver 16 hinterlegten Rekrutierungsparameter zumindest einer Studie maschinell abgeglichen wird. Insbesondere wird der Abgleichschritt 10 mittels des Abgleichprozesses durchgeführt. Zudem umfasst das Verfahren zur Patientenrekrutierung zumindest einen Bewertungsschritt 18, in welchem zumindest ein Eignungsparameter einer Eignung des Patienten 14 für die Studie in Abhängigkeit von dem Patientenparameter und dem Rekrutierungsparameter maschinell ermittelt und auf zumindest einem Server 20, insbesondere auf dem Patientenrekrutierungsserver 36, hinterlegt wird. Insbesondere wird der Bewertungsschritt 18 mittels des Bewertungsprozesses durchgeführt. Außerdem umfasst das Verfahren zur Patientenrekrutierung zumindest einen Zuordnungsschritt 22, in welchem zumindest eine Zuordnung zwischen dem Patienten 14 und der Studie in Abhängigkeit von dem Eignungsparameter durchgeführt wird und zumindest eine Zuordnungsinformation auf zumindest einem Server, insbesondere auf dem Patientenrekrutierungsserver 36, hinterlegt wird.

In dem Zuordnungsschritt 22 wird dem Patienten 14 die Studie in Abhängigkeit von dem Eignungsparameter zugeordnet. Insbesondere dient der Patient 14 als Ausgangspunkt zur Suche geeigneter Studien, die diesem bei Eignung zugeordnet werden.

In dem Zuordnungsschritt 22 werden dem Patienten 14 mehrere geeignete Studien zugeordnet. Insbesondere ist auf dem Patientenrekrutierungsserver 36 zu mehreren oder allen in der Gesundheitsinstitution 50 befindlichen Patienten 14 jeweils eine Anzahl geeigneter Studien, die diesen zugeordnet sind, hinterlegt. Vorzugsweise ist auf dem Patientenrekrutierungsserver 36 für jeden Patienten 14 eine Information hinterlegt, die angibt, für welche Studien, die insbesondere an der Gesundheitsinstitution 50 durchgeführt werden, der Patient 14 grundsätzlich geeignet oder zumindest teilweise geeignet und/oder zumindest nicht ungeeignet erscheint.

Im vorliegenden Fall wird dem Patienten 14 in dem Zuordnungsschritt 22 eine geordnete Liste geeigneter Studien zugeordnet, welche auf zumindest einem Server 20, insbesondere auf dem Patientenrekrutierungsserver 36 hinterlegt wird. Vorzugsweise ist für mehrere oder alle in der Gesundheitsinstitution 50 befindlichen Patienten 14 jeweils eine individuelle geordnete Liste geeigneter Studien auf dem Patientenrekrutierungsserver 36 hinterlegt. Es ist denkbar, dass für jeden Patienten 14, der sich aktuell in der Gesundheitsinstitution 50 befindet, eine entsprechende geordnete Liste automatisiert erstellt wird, beispielsweise erstmalig bei dessen Registrierung und/oder Einlieferung und/oder Aufnahme. Es ist auch denkbar, dass eine geordnete Liste geeigneter Studien erst auf zumindest eine Freigabe hin erstellt wird, die beispielsweise von klinischem Personal 30 und/oder vom Patienten 14 selbst gegeben wird. Beispielsweise wäre denkbar, dass der Patient 14 bei seiner Aufnahme zustimmen muss, dass seine Eignung für Studien grundsätzlich vom Patientenrekrutierungsserver 36 überprüft werden darf.

Die geordnete Liste wird nach zumindest einem maschinell ermittelten und/oder nach zumindest einem vorgebbaren Kriterium geordnet maschinell erstellt, wie insbesondere auch oben angegeben. Erfindungsgemäß wird die geordnete Liste nach einem Match-Score des Patienten 14 für die Studien der Liste geordnet, wobei vorteilhaft eine am besten geeignete Liste einen ersten oder einen letzten Eintrag der Liste bildet. Die Liste kann bedarfsweise auch nach einem Datum, beispielsweise einem Startdatum der entsprechenden Studien, nach einem Ort, an welchem die Studien durchgeführt werden, nach einer Studiendauer und/oder dergleichen geordnet werden. Insbesondere ist denkbar, dass die Liste in Abhängigkeit von zumindest einer Eingabe des Patienten 14 und/oder des klinischen Personals 30 geordnet wird. Insbesondere kann der Patient 14 mittels Eingabe einer bestimmten Präferenz eine entsprechend geordnete Liste erzeugen lassen.

In zumindest einem Auswahlschritt 24 wird eine Liste von abzugleichenden Studien erstellt. Insbesondere wird die Liste von abzugleichenden Studien in Abhängigkeit von zumindest einer dem Patienten 14 und/oder der Studie zugeordneten Standortinformation erstellt. Ferner ist denkbar, dass die Liste von abzugleichenden Studien in Abhängigkeit von einer Aufenthaltsdauer und/oder einem Aufenthaltsdatum des Patienten 14 und/oder einem Startdatum und/oder einer Studiendauer der Studie oder dergleichen erstellt wird. Beispielsweise ist denkbar, dass lediglich Studien auf die Liste abzugleichender Studien gesetzt werden, die an der Gesundheitsinstitution 50 während des Aufenthalts des Patienten 14 an der Gesundheitsinstitution 50 begonnen und/oder durchgeführt werden. Es ist aber auch denkbar, dass Studien auf die Liste abzugleichender Studien gesetzt werden, die an anderen Gesundheitsinstitutionen durchgeführt werden, beispielsweise zu Zwecken einer Vermittlung des Patienten.

Das Patientenrekrutierungssystem 34 weist eine mit dem Patientenrekrutierungsserver 36 verbindbare Ein- und/oder Ausgabeeinheit 28 auf, die zur Eingabe zumindest eines Patientenparameters vorgesehen ist. Die Ein- und/oder Ausgabeeinheit 28 ist in der Figur 1 schematisch dargestellt. Im vorliegenden Fall ist die Ein- und/oder Ausgabeeinheit 28 eine Ein- und Ausgabeeinheit zur Eingabe von Information, die beispielsweise auf dem Patientenrekrutierungsserver 36 hinterlegt werden kann, und zur Ausgabe von Information, beispielsweise von Studiendaten und/oder Patientendaten und/oder Eignungsparametern, insbesondere für den Patienten 14 und/oder für das klinische Personal 30. Ferner ist die Ein- und/oder Ausgabeeinheit 28 zur Eingabe zumindest einer Teilnahmeabsicht, insbesondere einer Teilnahmeabsicht des Patienten 14 an der Studie, vorgesehen. Zudem ist die Ein- und/oder Ausgabeeinheit 28 im vorliegenden Fall zur Ausgabe zumindest einer Studieninformation vorgesehen.

Die Ein- und/oder Ausgabeeinheit 28 ist zu einer Anordnung in der Gesundheitsinstitution 50 vorgesehen. Insbesondere ist die Ein- und/oder Ausgabeeinheit 28 zu einer Anordnung in zumindest einem Patientenzimmer 54 und/oder in zumindest einem Ärztezimmer 42 und/oder in zumindest einem Wartezimmer 44 vorgesehen.

Im vorliegenden Fall umfasst die Ein- und/oder Ausgabeeinheit 28 ein erstes Ein- und Ausgabegerät 56. Das erste Ein- und Ausgabegerät 56 ist in dem Patientenzimmer 54 angeordnet. Das erste Ein- und Ausgabegerät 56 kann beispielsweise an einem Patientenbett 112 und/oder an einer Wand und/oder an einer Decke des Patientenzimmers 54 insbesondere permanent befestigt und/oder installiert sein. Es ist auch denkbar, dass das Ein- und Ausgabegerät 56 als ein tragbares Ein- und Ausgabegerät ausgebildet ist, beispielsweise nach Art eines tragbaren Computers, eines Tablet-PCs, eines Kopfhörers, einer Datenbrille oder dergleichen. Der Patient 14 kann sich mittels des ersten Ein- und Ausgabegeräts 56 über, insbesondere an der Gesundheitsinstitution 50 laufende, Studien, insbesondere Studien der ihm zugeordneten Liste geeigneter Studien, informieren. Die Ein- und/oder Ausgabeeinheit 28 umfasst zumindest ein in dem Patientenzimmer 54 angeordnetes Display 46. Im vorliegenden Fall weist das erste Ein- und Ausgabegerät 56 das Display 46 auf. Das Display 46 kann als ein Touch-Display ausgebildet sein. Insbesondere ist das Display 46 zu einer Eingabe und/oder zu einer Ausgabe von Informationen vorgesehen, insbesondere von Studiendaten und/oder Eignungsdaten und/oder Patientendaten.

Ferner umfasst die Ein- und/oder Ausgabeeinheit 28 im vorliegenden Fall ein zweites Ein- und Ausgabegerät 58, das dem klinischen Personal 30 zugeordnet ist. Das zweite Ein- und Ausgabegerät 58 kann sich hinsichtlich einer Funktionalität und/oder hinsichtlich einer Ausgestaltung und/oder hinsichtlich mit demselben durchführbarer Aktionen unterscheiden oder auch mit diesem identisch sein. Im vorliegenden Fall ist das zweite Ein- und Ausgabegerät 58 beispielsweise nach Art eines Tablet-PCs oder eines elektronischen Papiers oder dergleichen ausgebildet und kann vorteilhaft von dem klinischen Personal 30 an unterschiedlichen Orten verwendet werden, beispielweise bei einer Visite. Hierbei ist insbesondere denkbar, dass das zweite Ein- und Ausgabegerät 58 zumindest einen Sensor aufweist, der zu einer automatisierten Erfassung einer Raumnummer und/oder zu einer Erkennung von Patienten 14 vorgesehen ist. Insbesondere ist denkbar, dass dem klinischen Personal 30 Informationen in Abhängigkeit von einer Erkennung des Sensors bereitgestellt werden, beispielsweise bei einem Betreten des Patientenzimmers 54 des Patienten 14 die dem Patienten 14 zugeordnete Liste geeigneter Studien.

Ferner umfasst die Ein- und/oder Ausgabeeinheit 28 im vorliegenden Fall ein drittes Ein- und Ausgabegerät 60. Das dritte Ein- und Ausgabegerät 60 kann beispielsweise in einem Ärztezimmer 42 der Gesundheitsinstitution 50 angeordnet sein. Im vorliegenden Fall ist das dritte Ein- und Ausgabegerät 60 als ein fest installiertes Touch-Display ausgebildet, über welches Studiendaten und/oder Patientendaten und/oder geordnete Listen geeigneter Studien für unterschiedliche Patienten 14 abgerufen werden können. Insbesondere ist denkbar, dass das dritte Ein- und Ausgabegerät 60 dazu vorgesehen ist, insbesondere unaufgefordert zumindest eine Benachrichtigung auszugeben, beispielsweise, wenn in der Gesundheitsinstitution 50 ein Patient 14 aufgenommen wurde, der einen hohen Match-Score mit einer bestimmten Studie aufweist.

Zudem umfasst die Ein- und/oder Ausgabeeinheit 28 im vorliegenden Fall ein viertes Ein- und Ausgabegerät 62. Das vierte Ein- und Ausgabegerät 62 kann beispielsweise in einem Wartezimmer 44 angeordnet sein. Das vierte Ein- und Ausgabegerät 62 kann dazu vorgesehen sein, einem Patienten 14, beispielsweise während einer Wartezeit, Studiendaten anzuzeigen, insbesondere auf eine Registrierung des Patienten 14 hin, die ein Abrufen seines Patientendatensatzes von dem Patientendatenserver 12 erlaubt.

Zudem ist denkbar, dass die Ein- und/oder Ausgabeeinheit 28 zumindest eine mobile Vorrichtung 48 umfasst. Im vorliegenden Fall umfasst die Ein- und/oder Ausgabeeinheit 28 zumindest eine mobile Vorrichtung 48 des klinischen Personals 30, beispielsweise ein Dienst-Smartphone. Die mobile Vorrichtung 48 bildet vorteilhaft zumindest ein Ein- und Ausgabegerät der Ein- und/oder Ausgabeeinheit 28 aus. Die mobile Vorrichtung 48 kann beispielsweise zur Ausführung zumindest eines Programms, insbesondere einer App, vorgesehen sein, die eine Einbindung in das Patientenrekrutierungssystem 34 ermöglicht. Beispielsweise kann die mobile Vorrichtung 48 auch als ein Tablet-PC, eine Smartwatch, ein Smart Cloth, ein Smartband, ein beliebiges anderes Wearable, ein Computer oder dergleichen mehr sein. Zudem ist denkbar, dass die mobile Vorrichtung 48 eine mobile Vorrichtung des Patienten 14 ist.

Im Folgenden wird nochmals auf die Figur 2 verwiesen. In zumindest einem Ausgabeschritt 26 wird mittels zumindest einer Ein- und/oder Ausgabeeinheit 28 zumindest eine Studieninformation ausgegeben. Die Studieninformation wird beispielsweise dem Patienten 14 angezeigt. Alternativ oder zusätzlich ist denkbar, dass die Studieninformation dem klinischen Personal 30 angezeigt wird. Vorzugsweise wird die Studieninformation anhand zumindest eines auf dem Studiendatenserver 16 hinterlegten Studiendatensatzes generiert, beispielsweise unter Verwendung einer Studienbeschreibung und/oder einer Kontaktinformation und/oder einer Standortinformation und/oder einer Studiendauer oder dergleichen. Dabei ist denkbar, dass dem Patienten 14 eine andere Studieninformation ausgegeben wird als dem klinischen Personal 30. Insbesondere wird die Studieninformation für den Patienten 14 patientengerecht aufbereitet, beispielsweise im Hinblick auf eine Sprache und/oder eine Ausführlichkeit und/oder einer Verwendung weiterführender Hintergrundinformation, die beispielsweise der Patient 14 benötigt, die für klinisches Personal 30 aber beispielsweise allgemeines Fachwissen darstellt.

Zudem wird in zumindest einem Verfahrensschritt 64 mittels der Ein- und/oder Ausgabeeinheit 28 zumindest ein Teilnahmeabsichtsparameter des Patienten 14 eingegeben und auf zumindest einem Server, insbesondere auf dem Patientenrekrutierungsserver 36 hinterlegt. Insbesondere ist der Teilnahmeabsichtsparameter ein der Studie zugeordneter Teilnahmeabsichtsparameter. Der Teilnahmeabsichtsparameter kann dabei beispielsweise die Werte "Teilnahme beabsichtigt" oder "Teilnahme nicht beabsichtigt" annehmen. Zudem ist denkbar, dass der Teilnahmeabsichtsparameter anzeigt, dass der Patient 14 grundsätzlich an der Studie interessiert ist, aber beispielsweise zusätzliche Informationen, insbesondere bereitgestellt von einem Studienverantwortlichen und/oder von klinischem Personal 30, wünscht und/oder erneut an die Studie erinnert werden möchte, beispielsweise um Bedenkzeit zu erhalten. Es ist denkbar, dass dem Patienten 14 nach Eingabe des Teilnahmeabsichtsparameters die geordnete Liste geeigneter Studien um die Teilnahmeabsichtsinformation ergänzt angezeigt werden kann, sodass der Patient 14 einer Anzeige die Information entnehmen kann, an welchen Studien er teilzunehmen beabsichtigt.

Ferner wird zumindest ein Ausgabeparameter für die Ausgabe der Studieninformation, insbesondere in dem Ausgabeschritt 26, in Abhängigkeit von zumindest einem Patientenmerkmal, insbesondere von zumindest einem Patientenparameter, angepasst.

Beispielsweise ist denkbar, dass der Patient 14 mittels Eingaben, die er insbesondere mit dem ihm zugeordneten ersten Ein- und Ausgabegerät 56 vornimmt, eine Art und/oder einen Umfang von dargestellter Information anpassen kann. Beispielsweise ist denkbar, dass dem Patienten 14 zunächst die geordnete Liste geeigneter Studien angezeigt wird und dieser anschließend, beispielsweise durch Antippen und/oder Anklicken oder Auswählen detailliertere Studieninformationen zu einzelnen Studien abrufen kann. Ferner ist denkbar, dass zu einer bestimmten Studie unterschiedliche Studieninformationen, beispielsweise für unterschiedliche Zielgruppen, hinterlegt sind. Es ist dann denkbar, dass eine Art und/oder ein Umfang einer Darstellung von Studieninformationen und/oder ein Wording und/oder zusätzliche externe Links oder dergleichen in Abhängigkeit von einem oder mehreren Patientenparametern, insbesondere automatisiert, angepasst wird, vorzugsweise unabhängig von einer Patienteneingabe, beispielsweise in Abhängigkeit von dessen Muttersprache und/oder Geschlecht und/oder Alter und/oder einer etwaigen Sehbeeinträchtigung oder dergleichen.

Außerdem kann zumindest eine Kontaktinformation zu der Studie für den Patienten 14 bereitgestellt werden. Beispielsweise kann ein Name und/oder eine Telefonnummer und/oder ein Standort eines Studienverantwortlichen angezeigt werden. Es ist ferner denkbar, dass die Ein- und/oder Ausgabeeinheit 28 dazu vorgesehen ist, zumindest einen Kommunikationskanal, beispielsweise eine Gesprächsleitung und/oder eine Videogesprächsleitung und/oder einen Textnachrichtenkanal oder dergleichen bereitzustellen, der vorzugsweise zu einer Kontaktaufnahme mit einem Studienverantwortlichen durch den Patienten 14 vorgesehen ist.

Insbesondere in dem Bewertungsschritt 18 wird zumindest eine hierarchische Bewertungsliste mit mehreren Bewertungskriterien anhand mehrerer Rekrutierungsparameter der Studie maschinell erstellt, anhand derer der Eignungsparameter ermittelt wird. Die Bewertungsliste kann auch, insbesondere als Ganzes, vorab, insbesondere automatisiert, erstellt und vorteilhaft auf dem Patientenrekrutierungsserver 36 und/oder auf dem Studiendatenserver 16 hinterlegt worden sein. Dabei ist denkbar, dass die Bewertungsliste unter Verwendung zumindest einer semantischen Analyse zumindest einer textuellen Beschreibung, insbesondere einer Studienbeschreibung, zumindest teilweise automatisiert erstellt wird. Es ist auch denkbar, dass die Ein- und/oder Ausgabeeinheit 28 zu einer Eingabe und/oder einer Unterstützung, insbesondere eines Studienverantwortlichen, bei einer Erstellung der Bewertungsliste vorgesehen ist.

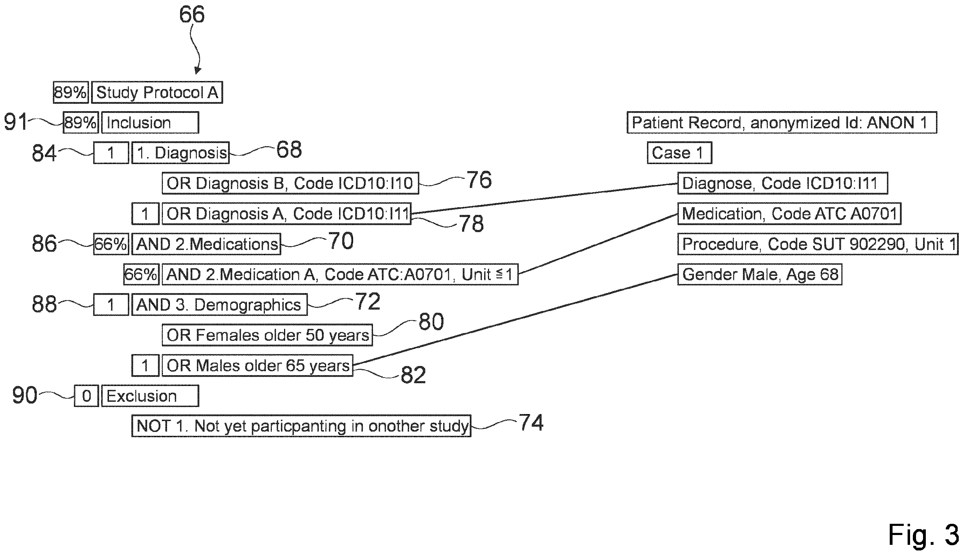

Die Figur 3 zeigt eine schematische Darstellung einer exemplarischen Bewertungsliste 66 einer exemplarischen Studie. Die exemplarische Bewertungsliste 66 basiert auf einem bestimmten Studienprotokoll.

Die exemplarische Bewertungsliste 66 umfasst mehrere Bewertungskriterien 68, 70, 72, die potentiell zu einem Einschluss eines Patienten 14 führen können. Im vorliegenden Fall ist ein erstes Bewertungskriterium 68 ein Diagnosekriterium. Gemäß Studienprotokoll sollte für einen Patienten 14, der für die Studie potentiell geeignet sein könnte, eines von zwei möglichen Diagnosekriterien 76, 78 erfüllt sein, das heißt eine von zwei möglichen Diagnosen sollte vorliegen. Der Patient 14 erfüllt im vorliegenden Fall beispielsweise die erste Diagnose. Dem ersten Bewertungskriterium wird daher ein Teil-Match-Score 84 von 1 zugeordnet.

Ein zweites Bewertungskriterium 70 ist im vorliegenden Fall eine Medikation. Der Patient 14 erfüllt im vorliegenden Fall dieses Kriterium teilweise, beispielsweise, da er ein ähnliches Medikament wie ein gemäß dem zweiten Bewertungskriterium 70 gefordertes einnimmt. Dem zweiten Bewertungskriterium 70 wird daher ein Teil-Match-Score 86 beispielsweise von 0,66 zugeordnet.

Ein drittes Bewertungskriterium 72 ist im vorliegenden Fall beispielsweise ein demographisches Kriterium. Gemäß Studienprotokoll sollte ein Patient 14, der für die Studie potentiell geeignet sein könnte, eines von zwei möglichen demographischen Kriterien 80, 82 erfüllen. Beispielsweise sollte ein Patient 14 weiblich und älter als 50 Jahre oder männlich und älter als 65 Jahre sein. Im vorliegenden Fall ist der Patient beispielsweise männlich und 68 Jahre alt. Dem dritten Bewertungskriterium 72 wird daher ein Teil-Match-Score 88 von 1 zugeordnet.

Zudem umfasst die Bewertungsliste 66 im vorliegenden Fall ein viertes Bewertungskriterium 74, das potentiell zu einem Ausschluss eines Patienten 14 führen kann. Das vierte Bewertungskriterium 74 ist im vorliegenden Fall ein Ausschlusskriterium. Das vierte Bewertungskriterium 74 führt im vorliegenden Fall beispielsweise zu einem Ausschluss eines Patienten 14, falls dieser bereits an einer anderen Studie teilnimmt. Im vorliegenden Fall nimmt der Patient 14 beispielsweise aktuell an keiner anderen Studie teil. Dem vierten Bewertungskriterium 74 wird daher ein Wert 90 von 0 zugeordnet. Es ist denkbar, dass eine Erfüllung eines Ausschlusskriteriums zu einem Ausschluss eines Patienten 14 führt, unabhängig von einer Bewertung anderer Bewertungskriterien.

Im Fall der exemplarischen Bewertungsliste 66 wird den Bewertungskriterien 68, 70, 72, 74 jeweils ein Gewichtungsparameter zugeordnet. Für das erste, zweite und dritte Bewertungskriterium 68, 70, 72 ist im vorliegenden Fall der Gewichtungsfaktor jeweils 1. Insbesondere deshalb wird der Gesamt-Match-Score als arithmetisches Mittel gebildet. Für unterschiedliche Gewichtungsparameter könnte beispielsweise ein gewichteter Mittelwert gebildet werden. Es ist auch denkbar, dass die Gewichtungsparameter eine hierarchische Bewertung angeben, sodass beispielsweise bestimmte Teil-Match-Scores für die Gesamtbewertung unberücksichtigt bleiben, falls andere Teil-Match-Scores in bestimmten Bereichen liegen, beispielsweise bestimmte Grenzwerte unter- und/oder überschreiten. Ferner wird im vorliegenden Fall dem vierten Bewertungskriterium 74 ein Gewichtungsparameter derart zugeordnet, das ein Gesamt-Match-Score von 0 erzwungen wird, falls das vierte Bewertungskriterium 74 erfüllt ist, der Patient 14 also beispielsweise bereits an einer anderen Studie teilnimmt. Gewichtungsparameter können entsprechend beispielsweise, insbesondere diskrete und/oder stetige Funktionen und/oder Berechnungsvorschriften und/oder Zahlenwerte und/oder Kategorisierungen und dergleichen enthalten.

Im vorliegenden Fall wird ein Gesamt-Match-Score 91 des Patienten 14 für die exemplarische Studie ermittelt, beispielsweise als arithmetisches Mittel von Teil-Match-Scores, sofern kein Ausschlusskriterium erfüllt ist. Im vorliegenden Fall bestimmt sich der Gesamt-Match-Score für den Patienten 14 demnach beispielsweise zu 0,89. Der Gesamt-Match-Score 91 und die entsprechende Zuordnung zwischen dem Patienten 14 und der exemplarischen Studie werden auf dem Patientenrekrutierungsserver 36 hinterlegt.

Selbstverständlich sind beliebige andere Bewertungslisten denkbar, die eine andere Anzahl von Kriterien und/oder Hierarchieebenen etc. umfassen. Zudem ist anstelle eines arithmetischen Mittels denkbar, dass Bewertungskriterien unterschiedlich stark gewichtet werden.

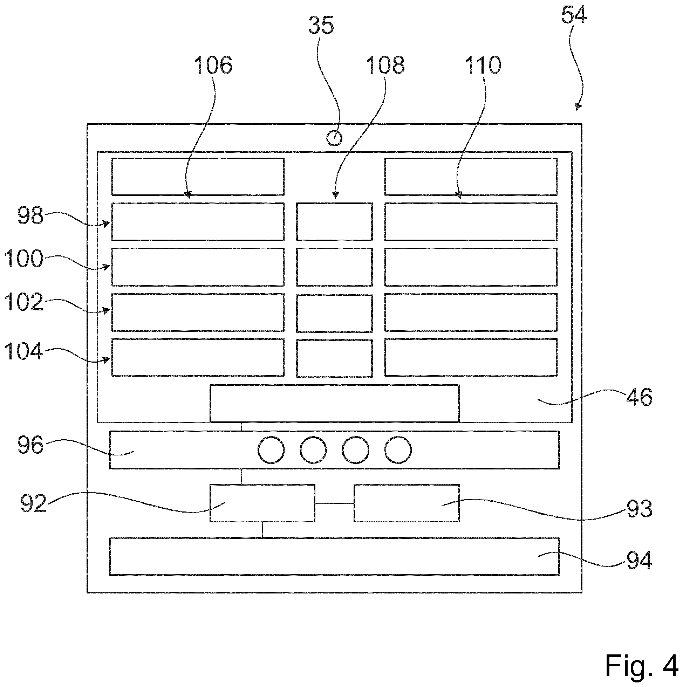

Insbesondere analog zu dem Fall der exemplarischen Studie werden die Liste geeigneter Studien sowie zugehörige Eignungsparameter ermittelt und auf dem Patientenrekrutierungsserver 36 hinterlegt. Wie oben erwähnt, kann diese Liste mittels der Ein- und/oder Ausgabeeinheit 28, insbesondere mittels des dem Patienten 14 zugeordneten ersten Ein- und Ausgabegeräts 56, dem Patienten 14 zur Verfügung gestellt werden. Die Figur 4 zeigt eine schematische Darstellung des ersten Ein- und Ausgabegeräts 56. Das Ein- und Ausgabegerät 56 weist das Display 46 auf. Zudem weist das Ein- und Ausgabegerät 56 eine Recheneinheit 92 und einen internen Speicher 93auf. Außerdem weist das Ein- und Ausgabegerät 56 eine Schnittstelle 94 zur Verbindung mit einem Netzwerk, insbesondere zur Verbindung mit dem Patientenrekrutierungsserver 36 auf. Ferner weist das Ein- und Ausgabegerät 56 eine Eingabeeinheit 96 auf, die beispielsweise mehrere Knöpfe umfasst. Es ist auch denkbar, dass die Eingabeeinheit 96 eine Maus und/oder eine Tastatur umfasst. Zudem ist denkbar, dass die Eingabeeinheit 96 zumindest teilweise von dem Display 46 gebildet ist, beispielsweise, falls dieses als Touch-Display ausgebildet ist.

In zumindest einem Betriebszustand des Ein- und Ausgabegeräts 56 wird dem Patienten 14 Information zu wenigstens einem Teil der Studien der Liste geeigneter Studien angezeigt, beispielsweise zu den Studien mit den höchsten Match-Scores. Im dargestellten Fall, der selbstverständlich rein exemplarisch zu verstehen ist, werden beispielsweise zu vier exemplarischen Studien 98, 100, 102, 104 jeweils ein Studientitel (Spalte 106), ein Match-Score (Spalte 108) sowie eine Kontaktinformation (Spalte 110) dargestellt. Beispielsweise durch Anklicken oder Antippen einer entsprechenden Zeile einer dargestellten Liste kann der Patient 14 weitere Studieninformationen abrufen und/oder eine Teilnahmeabsicht eingeben und/oder weitere Informationen, beispielsweise von dem klinischen Personal 30 und/oder von einem Studienverantwortlichen anfordern. Selbstverständlich sind beliebige andere Darstellungen und/oder Arten einer Informationsbereitstellung denkbar.

Im Folgenden wird nochmals auf die Figuren 1 und 2 Bezug genommen. Im vorliegenden Fall wird, beispielsweise in dem Abgleichschritt 10 und/oder in dem Bewertungsschritt 18 und/oder zu einem anderen geeigneten Zeitpunkt zumindest eine Liste von Bewertungskriterien erstellt, die zumindest eine Eingabe zumindest eines klinischen Personals 30 erfordern. Diese Liste von Bewertungskriterien umfasst insbesondere Bewertungskriterien, die nicht automatisiert ausgewertet werden können, beispielsweise aufgrund eines Nichtvorliegens entsprechender Patientenparameter und/oder aufgrund einer Notwendigkeit einer Eingabe einer ärztlichen Einschätzung oder dergleichen. Vorzugsweise wird das klinische Personal 30, beispielsweise mittels des zweiten Ein- und Ausgabegeräts 58, zu einer Eingabe entsprechender Informationen aufgefordert. Vorteilhaft werden entsprechende Eingaben des klinischen Personals 30 zu einer maschinellen Verarbeitung zumindest der betroffenen Bewertungskriterien verwendet und/oder auf dem Patientendatenserver 12 und/oder dem Studiendatenserver 16 und/oder dem Patientenrekrutierungsserver 36 hinterlegt.

Ferner wird in zumindest einem Eingabeschritt 32 mittels der Ein- und/oder Ausgabeeinheit 28 zumindest ein weiterer Patientenparameter abgefragt und/oder eingegeben. Hierbei kann es sich um einen Patientenparameter handeln, der gemäß der beschriebenen Liste von Bewertungskriterien benötigt wird. Vorteilhaft wird der weitere Patientenparameter von dem Patienten 14 abgefragt und/oder eingegeben. Beispielsweise kann der Patient 14 zu einer Vervollständigung seines Patientendatensatzes aufgefordert werden. Insbesondere werden in dem Eingabeschritt persönliche Daten und/oder Gesundheitsdaten und/oder Präferenzen des Patienten 14 oder dergleichen von dem Patienten 14 eingegeben und vorteilhaft zu einer Ergänzung eines dem Patienten 14 zugeordneten Patientendatensatzes verwendet.

Außerdem wird, beispielsweise in dem Ausgabeschritt 26, mittels der Ein- und/oder Ausgabeeinheit 28 zumindest eine Eignungsinformation des Patienten 14 für das dem Patienten 14 zugeordnete klinische Personal 30 zur Verfügung gestellt. Beispielsweise kann die Eignungsinformation mittels des zweiten Ein- und Ausgabegeräts 58 und/oder mittels des dritten Ein- und Ausgabegeräts 60 ausgegeben werden. Beispielsweise kann dem klinischen Personal 30 die Liste geeigneter Studien angezeigt werden. Insbesondere wird dem klinischen Personal 30 ermöglicht, den Patienten 14 gegebenenfalls auf dessen Eignung für eine bestimmte Studie aufmerksam zu machen. Grundsätzlich ist denkbar, dass das klinische Personal 30 analog zu dem Patienten 14 Studiendaten mittels der Ein- und/oder Ausgabeeinheit 28 abfragen und/oder Informationen eingeben kann.

Im vorliegenden Fall wird zudem zumindest ein Patientenparameter mittels zumindest einer Sensoreinheit 35 automatisiert erfasst und maschinell verarbeitet, beispielsweise in dem Eingabeschritt 32. Hierbei sind, wie oben erwähnt, beliebige Sensoren denkbar, die beispielsweise in dem Patientenzimmer 54 und/oder an einem Patientenbett 112 angebracht sind. Insbesondere kann es sich um ohnehin vorhandene Sensoren von medizinischen Überwachungsgeräten handeln, die beispielsweise in ein Netzwerk des Patientendatenservers 12 eingebunden werden können. Im vorliegenden Fall weist das dem Patienten 14 zugeordnete erste Ein- und Ausgabegerät 56 die Sensoreinheit 35 auf. Die Sensoreinheit 35 umfasst im vorliegenden Fall eine digitale Videokamera. Mittels der Sensoreinheit 35 können Bilder und Videos aufgenommen werden, die anschließend beispielsweise für eine Bildanalyse herangezogen werden können. Mittels der Sensoreinheit 35 können beispielsweise Bilder von Medikamentenverpackungen des Patienten 14 aufgenommen werden. Anhand einer Bildanalyse dieser Bilder wird dann eine Liste von Medikamenten des Patienten 14 erstellt, die dieser beispielsweise zu einer Korrektheitsprüfung und/oder Ergänzung angezeigt bekommt. Beispielsweise kann nach einer Erkennung eines bestimmten Medikaments abgefragt werden, seit wann und/oder wie häufig der Patient 14 besagtes Medikament einnimmt.

Zudem kann zumindest ein Ausgabeparameter für eine Ausgabe zumindest einer Studieninformation in Abhängigkeit von dem mittels der Sensoreinheit 35 erfassten Patientenparameter angepasst werden. Im vorliegenden Fall wird beispielsweise anhand der ermittelten Medikation des Patienten 14 dessen Vertrautheit mit bestimmten Krankheitsbildern automatisiert ermittelt. Ferner wird in Abhängigkeit von einer geschätzten Vertrautheit des Patienten 14 mit bestimmten Krankheitsbildern eine Ausführlichkeit von Studienbeschreibungen angepasst, beispielsweise um dem Patienten 14 zusätzliche Informationen zu Studien bereitzustellen, die für Ihn aufgrund seiner persönlichen Krankheitsgeschichte interessant sein könnten und ihn zu einer Teilnahme animieren. Wie oben erwähnt sind beliebige weitere Anpassungen von Ausgabeparametern denkbar. So kann beispielsweise ein Geschlecht und/oder ein Alter und/oder ein Verhaltensparameter und/oder eine Aufmerksamkeit des Patienten 14 oder dergleichen anhand zumindest einer Sensorerfassung ermittelt und ein Ausgabeparameter entsprechend angepasst werden.

## Patentansprüche

1. Verfahren zur Patientenrekrutierung für eine Studie, insbesondere für zumindest eine klinische Studie, wobei in zumindest einem Abgleichschritt (10) zumindest ein auf einem Patientendatenserver (12) hinterlegter Patientenparameter zumindest eines Patienten (14) mit zumindest einem auf einem Studiendatenserver (16) hinterlegten Rekrutierungsparameter zumindest einer Studie maschinell abgeglichen wird,
wobei
in zumindest einem Bewertungsschritt (18) zumindest ein, als ein Match-Score ausgebildeter Eignungsparameter einer Eignung des Patienten (14) für die Studie in Abhängigkeit von dem Patientenparameter und dem Rekrutierungsparameter maschinell ermittelt und auf zumindest einem Server (20), insbesondere einem Patientenrekrutierungsserver (36), hinterlegt wird, wobei der Match-Score mit einer Güte eines Matches zwischen dem Patienten (14) und der Studie korreliert,
wobei in zumindest einem Auswahlschritt (24) eine Liste von abzugleichenden Studien erstellt wird,
wobei die Liste abzugleichender Studien abgearbeitet wird, um eine geordnete Liste geeigneter Studien zu erstellen und dem Patienten (14) zuzuordnen, wobei in zumindest einem Zuordnungsschritt (22) zumindest eine Zuordnung zwischen dem Patienten (14) und der Studie in Abhängigkeit von dem Eignungsparameter durchgeführt wird und zumindest eine Zuordnungsinformation auf zumindest einem Server (20), insbesondere dem Patientenrekrutierungsserver (36), hinterlegt wird,
wobei in dem Zuordnungsschritt (22) dem Patienten (14) mehrere geeignete Studien in Abhängigkeit von jeweils entsprechenden Eignungsparametern zugeordnet werden,
wobei in dem Zuordnungsschritt (22) dem Patienten (14) die nach den jeweiligen Eignungsparametern geordnete Liste der geeigneten Studien zugeordnet wird, welche auf dem Server (20), insbesondere dem Patientenrekrutierungsserver (36), hinterlegt wird,
wobei dem Patienten (14) Studien zugeordnet werden, bei denen der Match-Score einen bestimmten Grenzwert über- oder unterschreitet,
wobei die geordnete Liste nach dem maschinell ermittelten Match-Score des Patienten (14) geordnet maschinell erstellt wird,
wobei Patientendaten grundsätzlich in anonymisierter und/oder pseudonymisierter Form zwischen dem Patientendatenserver (12), dem Studiendatenserver (16) und dem Patientenrekrutierungsserver (36) ausgetauscht werden,
wobei in zumindest einem Ausgabeschritt (26) mittels zumindest einer Ein- und/oder Ausgabeeinheit (28) Studieninformationen der Studien aus der geordneten Liste für welche der Patient (14) zumindest teilweise geeignet ist, in geordneter Form ausgegeben werden,
wobei die Ein- und/oder Ausgabeeinheit (28) ein Display (46) umfasst, welches in einem Patientenzimmer (54) einer Gesundheitsinstitution (50) angeordnet ist, wobei der Auswahlschritt (24), der Abgleichschritt (10) und der Zuordnungsschritt (22) automatisiert in bestimmten Zeitabständen und/oder nachdem zusätzliche Patientenparameter eingegeben worden sind, wiederholt durchgeführt wird, wodurch die Liste abzugleichender Studien in Echtzeit wiederholt aktualisiert und/oder in Echtzeit wiederholt abgearbeitet wird,
wobei zumindest ein Einmaligkeitsmerkmal des Patienten mit einer dazu vorgesehenen Sensoreinheit (35) erkannt wird und/oder ein nicht zwingend eindeutiges aber dem Patienten zugeordnetes Merkmal des Patienten, wie beispielsweise ein Körpergewicht, ein Geschlecht, ein geschätztes Alter, eine Augenfarbe und/oder dergleichen, mit hinterlegten und/oder mit aktuellen Erfassungen der Sensoreinheit (35) verglichen wird, um den Patienten zu erkennen,
und wobei das Display (46) und/oder ein anderes Ausgabemittel der Ein- und/oder Ausgabeeinheit (28) zumindest vorübergehend deaktiviert und/oder gesperrt wird, wenn von der Sensoreinheit (35) ein von dem Patienten abweichender anderer Patient und/oder kein Patient erkannt wird.

2. Verfahren nach Anspruch 1, **dadurch gekennzeichnet, dass** in dem Auswahlschritt (24) die Liste von abzugleichenden Studien in Abhängigkeit von zumindest einer dem Patienten (14) und/oder der Studie zugeordneten Standortinformation erstellt wird.

3. Verfahren nach Anspruch 1 oder 2, **dadurch gekennzeichnet, dass** mittels der Ein- und/oder Ausgabeeinheit (28) zumindest ein Teilnahmeabsichtsparameter des Patienten (14) eingegeben und auf zumindest einem Server (20), insbesondere dem Patientenrekrutierungsserver (36), hinterlegt wird.

4. Verfahren nach einem der vorhergehenden Ansprüche, **dadurch gekennzeichnet, dass** zumindest eine hierarchische Bewertungsliste (66) mit mehreren Bewertungskriterien anhand mehrerer Rekrutierungsparameter der Studie maschinell erstellt wird, anhand derer der Eignungsparameter ermittelt wird, wobei vorzugsweise den Bewertungskriterien jeweils zumindest ein Gewichtungsparameter zugeordnet wird.

5. Verfahren nach einem der vorhergehenden Ansprüche, **dadurch gekennzeichnet, dass** zumindest eine Kontaktinformation zu der Studie für den Patienten (14) bereitgestellt wird.

6. Verfahren nach einem der vorhergehenden Ansprüche, **dadurch gekennzeichnet, dass** in zumindest einem Eingabeschritt (32) mittels zumindest einer Ein- und/oder Ausgabeeinheit (28) zumindest ein weiteres Patientenparameter abgefragt und/oder eingegeben wird.

7. Verfahren nach einem der vorhergehenden Ansprüche, **dadurch gekennzeichnet, dass** mittels zumindest einer Ein- und/oder Ausgabeeinheit (28) zumindest eine Eignungsinformation des Patienten (14) für ein dem Patienten (14) zugeordnetes klinisches Personal (30) zur Verfügung gestellt wird.

8. Patientenrekrutierungssystem (34) zur Rekrutierung von Patienten (14) für klinische Studien, welches zur Durchführung eines Verfahrens nach Anspruch 1 speziell ausgelegt ist,
wobei das Patientenrekrutierungssystem (34) einen Patientenrekrutierungsserver (36), einen Patientendatenserver (12) und einen Studiendatenserver (16) aufweist,
wobei eine Ein- und/oder Ausgabeeinheit (28) Teil des Patientenrekrutierungssystems (34) ist,
wobei die Ein- und/oder Ausgabeeinheit (28) zumindest ein in einem Patientenzimmer (54) einer Gesundheitsinstitution (50) angeordnetes Display (46) umfasst, und
wobei das Patientenrekrutierungssystem (34) eine Sensoreinheit (35) aufweist.

## Claims

1. Method for recruiting patients for a study, in particular for at least one clinical study, wherein at least one matching step (10) involves at least one patient parameter of at least one patient (14), stored on a patient data server (12), being matched, in a computerized manner, with at least one recruitment parameter of at least one study, stored on a study data server (16),
wherein at least one rating step (18) involves at least one suitability parameter, realized as a match score, of a suitability of the patient (14) for the study being determined, in a computerized manner, as a function of the patient parameter and the recruitment parameter and stored on at least one server (20), in particular a patient recruitment server (36), wherein the match score is correlated with a quality of a match between the patient (14) and the study,
wherein at least one selection step (24) involves a list of studies to be matched being produced,
wherein the list of studies to be matched is worked through in order to produce an ordered list of suitable studies and preferably to associate said list with the patient (14),
wherein in at least one association step (22) at least an association between the patient (14) and the study is made as a function of the suitability parameter, and at least one piece of association information is stored on at least one server (20), in particular the patient recruitment server (36),
wherein the association step (22) involves a plurality of suitable studies being associated with the patient (14) as a function of respectively corresponding suitability parameters,
wherein the association step (22) involves an ordered list of the suitable studies being associated with the patient (14), said list being ordered with regard to the respective suitability parameters and stored on the server (20), in particular the patient recruitment server (36),
wherein studies in which the match score exceeds or goes below a certain limit value are associated with the patient (14),
wherein the ordered list is organized in a computerized manner according to the match score for the patient (14) that was determined in a computerized manner, wherein patient data are in principle exchanged between the patient data server (12), the study data server (16) and the patient recruitment server (36) in anonymized and/or pseudonymized form,
wherein at least one output step (26) involves study information of the studies from the ordered list, for which the patient (14) is at least partially suitable, being outputted in ordered form by means of at least one input and/or output unit (28), the input and/or output unit (28) comprising a display (46) that is arranged in a patient room (54) of a healthcare institution,
wherein the selection step (24), the matching step (10) and the association step (22) are performed repeatedly in automated fashion at specific time intervals and/or after additional patient parameters have been inputted,
such that the list of studies to be matched is repeatedly updated in real time, and/or repeatedly worked through in real time,
wherein by means of a sensor unit (35) intended for this purpose, at least one uniqueness feature of a patient is recognized
and/or a feature that is not necessarily unique but is associated with the patient, such as for example a body weight, a sex, an estimated age, an eye color and/or the like, is compared with stored and/or with current detections of the sensor unit (35) in order to identify the patient,
and wherein the display (46) and/or or another output means of the input and/or output unit (28) is at least temporarily deactivated and/or blocked if a different patient, other than the patient, and/or no patient is detected.

2. Method according to claim 1,
**characterized in that** the selection step (24) involves the list of studies to be matched being produced as a function of at least one piece of location information associated with the patient (14) and/or with the study.

3. Method according to claim 1 or 2,
**characterized in that** by means of the input and/or output unit (28) at least one participation intention parameter of the patient (14) and is inputted and is stored on at least one server (20), in particular the patient recruitment server (36).

4. Method according to one of the preceding claims,
**characterized in that** at least one hierarchic rating list (66) containing a plurality of rating criteria is produced in a computerized manner using a plurality of recruitment parameters of the study, on the basis of which rating list (66) the suitability parameter is determined,
wherein preferably respectively at least one weighting parameter is associated with each of the rating criteria.

5. Method according to one of the preceding claims,
**characterized in that** at least one piece of contact information pertaining to the study is provided for the patient (14).

6. Method according to one of the preceding claims,
**characterized in that** at least one input step (32) involves at least one input and/or output unit (28) being used to query and/or input at least one further patient parameter.

7. Method according to one of the preceding claims,
**characterized in that** at least one input and/or output unit (28) is used to make available at least one piece of suitability information of the patient (14) to clinical personnel (30) that is associated with the patient (14).

8. Patient recruitment system (34) for recruiting patients (14) for clinical studies, which is specifically designed to perform a method according to claim 1,
wherein the patient recruitment system (34) has a patient recruitment server (36), a patient data server (12) and a study data server (16),
wherein an input and/or output unit (28) is part of the patient recruitment system (34),
wherein the input and/or output unit (28) comprises at least one display (46), which is arranged in a patient room (54) of a healthcare institution (50), and
wherein the patient recruitment system (34) comprises a sensor unit (35).

## Revendications

1. Procédé de recrutement de patients pour une étude, en particulier pour au moins une étude clinique, où, dans au moins une étape de comparaison (10), au moins un paramètre-patient d'au moins un patient (14) qui est stocké sur un serveur de données de patient (12) est comparé par machine à au moins un paramètre-recrutement d'au moins une étude qui est stocké sur un serveur de données d'étude (16),
où
dans au moins une étape d'évaluation (18), au moins un paramètre d'aptitude d'une aptitude du patient (14) pour l'étude, réalisé comme score de correspondance, est déterminé par machine en fonction du paramètre-patient et du paramètre-recrutement et est stocké sur au moins un serveur (20), en particulier un serveur de recrutement de patients (36),
où le score de correspondance est corrélé avec une qualité d'une correspondance entre le patient (14) et l'étude,
où dans au moins une étape de sélection (24) une liste d'études à comparer est créée,
où la liste d'études à comparer est traitée point par point afin de créer une liste ordonnée d'études appropriées et de l'attribuer au patient (14),
où, dans au moins une étape d'attribution (22), au moins une attribution entre le patient (14) et l'étude est effectuée en fonction du paramètre d'aptitude et au moins une information d'attribution est stockée sur au moins un serveur (20), en particulier le serveur de recrutement de patients (36),
où, dans l'étape d'attribution (22), plusieurs études appropriées sont attribuées au patient (14) en fonction de paramètres d'aptitude respectivement correspondants,
où, dans l'étape d'attribution (22), la liste des études appropriées, ordonnée selon les paramètres d'aptitude respectifs, est attribuée au patient (14), ladite liste étant stockée sur le serveur (20), en particulier le serveur de recrutement de patients (36),
où des études sont attribuées au patient (14) pour lesquelles le score de correspondance est supérieur ou inférieur à une valeur limite spécifique,
où la liste ordonnée est créée par machine de manière ordonnée selon le score de correspondance du patient (14) qui a été déterminé par machine,
où des données de patient sont en principe échangées sous forme anonymisée et/ou pseudonymisée entre le serveur de données de patient (12), le serveur de données d'étude (16) et le serveur de recrutement de patients (36),
où dans au moins une étape de sortie (26), au moyen d'au moins une unité d'entrée et/ou de sortie (28), des informations d'étude des études de la liste ordonnée, pour lesquelles le patient (14) est au moins partiellement apte, sont sorties sous forme ordonnée,
où l'unité d'entrée et/ou de sortie (28) comprend un affichage (46) disposé dans une chambre de patient (54) d'un établissement de santé (50),
où l'étape de sélection (24), l'étape de comparaison (10) et l'étape d'attribution (22) sont effectuées à plusieurs reprises de manière automatisée à des intervalles de temps spécifiques et/ou après que des paramètres-patients supplémentaires ont été entrés, moyennant quoi la liste d'études à comparer est actualisée à plusieurs reprises en temps réel et/ou est traitée point par point à plusieurs reprises en temps réel,
où, moyennant une unité de capteur (35) pourvue à cette fin, au moins une caractéristique unique du patient est reconnue et/ou une caractéristique du patient non nécessairement unique mais attribuée au patient, comme par exemple un poids corporel, un sexe, un âge estimé, une couleur des yeux et/ou similaire, est comparée à des enregistrements stockés et/ou à des enregistrements actuels de l'unité de capteur (35) pour reconnaître le patient,
et où l'affichage (46) et/ou un autre moyen de sortie de l'unité d'entrée et/ou de sortie (28) est au moins temporairement désactivé et/ou bloqué si un autre patient différent du patient est reconnu par l'unité du capteur (35) et/ou si aucun patient n'est reconnu par l'unité de capteur (35).

2. Procédé selon la revendication 1,
**caractérisé en ce que** dans l'étape de sélection (24) la liste d'études à comparer est créée en fonction d'au moins une information de localisation attribuée au patient (14) et/ou à l'étude.

3. Procédé selon la revendication 1 ou 2,
**caractérisé en ce que** moyennant l'unité d'entrée et/ou de sortie (28) au moins un paramètre d'intention de participation du patient (14) est entré et est stocké sur au moins un serveur (20), en particulier le serveur de recrutement de patients (36).

4. Procédé selon l'une des revendications précédentes,
**caractérisé en ce qu'**au moins une liste d'évaluation hiérarchique (66) avec plusieurs critères d'évaluation est créée par machine à l'aide de plusieurs paramètres-recrutement de l'étude, sur la base de laquelle le paramètre d'aptitude est déterminé, où de préférence respectivement au moins un paramètre de pondération est attribué aux critères d'évaluation.

5. Procédé selon l'une des revendications précédentes,
**caractérisé en ce qu'**au moins une information de contact concernant l'étude est mise à disposition pour le patient (14).

6. Procédé selon l'une des revendications précédentes,
**caractérisé en ce que** dans au moins une étape d'entrée (32) au moins un autre paramètre du patient est extrait et/ou entré moyennant au moins une unité d'entrée et/ou de sortie (28).

7. Procédé selon l'une des revendications précédentes,
**caractérisé en ce que** moyennant au moins une unité d'entrée et/ou de sortie (28), au moins une information d'aptitude du patient (14) est mise à disposition pour un personnel clinique (30) attribué au patient (14).

8. Système de recrutement de patients (34) pour le recrutement de patients (14) pour des études cliniques, qui est spécialement conçu pour la mise en oeuvre d'un procédé selon la revendication 1,
où le système de recrutement de patients (34) comprend un serveur de recrutement de patients (36), un serveur de données de patient (12) et un serveur de données d'étude (16),
où une unité d'entrée et/ou de sortie (28) fait partie du système de recrutement de patients (34),
où l'unité d'entrée et/ou de sortie (28) comprend au moins un affichage (46) disposé dans une chambre de patient (54) d'un établissement de santé (50), et où le système de recrutement de patients (34) comprend une unité de capteur (35).
